# EUROPEAN PATENT APPLICATION

(11) **EP 4 137 562 A1**
(43) Date of publication of application: **22.02.2023**
(21) Application number: 22187395.3
(22) Date of filing: 04.05.2018
(51) Int. Cl.: C12N 5/0775

(54) **MESENCHYMAL LINEAGE PRECURSOR OR STEM CELLS WITH ENHANCED IMMUNOSUPPRESSION**

(30) Priority: 04.05.2017 AU 2017901633; 04.05.2017 AU 2017901636; 21.02.2018 AU 2018900551
(62) Divisional of application: 18726345.4
(71) Applicant: Mesoblast International Sàrl, 1217 Meyrin (CH)
(72) Inventor: ITESCU, Silviu, Melbourne 3000 (AU); SIMMONS, Paul, Melbourne 3000 (AU)
(74) Representative: Thomann, William John

(57) **Abstract**

The present disclosure relates to cellular therapy products comprising mesenchymal lineage precursor or stem cells and potency assay for these products. The present disclosure also relates to methods for treatment of immune or inflammatory disorders, and treatment or prevention of graft versus host disease (GVHD), or one or more symptoms associated with GVHD, by administration of mesenchymal lineage precursor or stem cells.

## Description

All documents cited or referenced herein, and all documents cited or referenced in herein cited documents, together with any manufacturer's instructions, descriptions, product specifications, and product sheets for any products mentioned herein or in any document incorporated by reference herein, are hereby incorporated herein by reference in their entirety.

The present disclosure claims priority from Australian provisional application AU 2017901633 entitled "Potency assay for immunosuppression" filed 4 May 2017; Australian provisional application AU 2017901636 entitled "Method for treating Graft versus Host Disease (GVDH)" filed 4 May 2017; and Australian provisional patent application AU 2018900551 entitled "Potency assay for immunosuppression II" filed 21 February 2018. The entire contents of these documents are hereby incorporated herein by reference in their entirety.

### Technical Field

The present disclosure relates to cellular therapy products comprising mesenchymal lineage precursor or stem cells and potency assay for these products. The present disclosure also relates to methods for treatment of immune or inflammatory disorders, and treatment or prevention of graft versus host disease (GVHD), or one or more symptoms associated with GVHD, by administration of mesenchymal lineage precursor or stem cells.

### Background

Several cellular therapy products for regenerative or immune therapy applications have advanced to clinical evaluation and market authorization. However, release of these cellular therapy products onto the market is hindered by their complexity and heterogeneity, which makes identification of relevant biologic activities and thus, definition of consistent cellular therapy product quality difficult.

Physiochemical parameters (for example, characterization of size, morphology, light-scattering properties, tensile strength, cell number, confluence, identification of phenotypic markers, secreted substances, genotype, gene expression profile) are routinely used for identification and quantification of the active substance, intermediates, impurities and contaminants. However, physiochemical parameters cannot confirm that a product will be biologically active and potent (i.e., elicit the desired effect). In contrast, biologic characterization takes into account the effect of the product on biologic systems, either modelled *in vitro* or *in vivo* in animals and ultimately in the clinic.

Pharmaceutical legislation in the United States and Europe requires that active substances whose molecular structure cannot be fully defined be evaluated for their potency before release onto the market. It is a legal requirement to evaluate the potency of each batch of a licensed cellular therapy product.

Potency testing must demonstrate the relevant biologic activity or activities of the product. It is not a requirement for potency testing to reflect all of the product's biological functions, but it should indicate one or more relevant biological functions. It is expected that accuracy, sensitivity, specificity and reproducibility will be established for the analytic methods used in potency testing and that they be suitably robust.

There is a need to develop products with improved potency for treatment of diseases where immunosuppression is desired. It is also preferable to identify parameters that are critical to the efficacy of cellular therapy products and to control them (e.g., via potency testing) such that products of consistent quality can be manufactured.

### Summary of the disclosure

The Applicant has developed an off-the-shelf *ex vivo* expanded allogeneic mesenchymal lineage precursor or stem cell (MLPSC) product which has improved immunosuppressive activity.

The present disclosure provides a composition comprising mesenchymal lineage precursor or stem cells or progeny thereof, wherein the mesenchymal lineage precursor or stem cells are cryopreserved and, after thawing, inhibit proliferation of activated T cells in a sample of PBMC by at least about 65%.

In one embodiment, the inhibition is measured by co-incubation of mesenchymal lineage precursor or stem cells with PBMC at a ratio of 1 mesenchymal lineage precursor or stem cell:5 PBMC, or less. For example, 1:10, 1:20, 1:30, 1:40, 1:50, 1:60, 1:70 1:80, 1:90, or 1 mesenchymal lineage precursor or stem cell: 100 PBMC, or less.

In one embodiment, co-incubation of mesenchymal lineage precursor or stem cells with PBMC at a ratio of 1 mesenchymal lineage precursor or stem cell:5 PBMC, or less, inhibits T cell proliferation by at least about 65%, at least about 70%, at least about 75%, at least about 80%, at least about 85%, or at least about 90%.

In one embodiment, the inhibition of proliferation of activated T cells is measured by inhibition of IL-2R 2Rα expression in the activated T cells.

In one embodiment, the mesenchymal lineage precursor or stem cell composition expresses TNFR1 in an amount of at least 110 pg/ml. For example, the mesenchymal lineage precursor or stem cell composition expresses TNFR1 in an amount of at least 150 pg/ml, or at least 200 pg/ ml, or at least 250 pg/ml, or at least 300 pg/ml, or at least 320 pg/ml, or at least 330 pg/ml, or at least 340 pg/ml, or at least 350 pg/ml.

In one embodiment, the mesenchymal lineage precursor or stem cells express TNFR1 in an amount of at least 13 pg/10⁶ cells. For example, the mesenchymal lineage precursor or stem cells express TNFR1 in an amount of at least 15 pg/10⁶ cells, or at least 20 pg/10⁶ cells, or at least 25 pg/10⁶ cells, or at least 30 pg/10⁶ cells, or at least 35 pg/10⁶ cells, or at least 40 pg/10⁶ cells, or at least 45 pg/10⁶ cells, or at least 50 pg/10⁶ cells.

In one embodiment, the mesenchymal lineage precursor or stem cells are isolated by immunoselection and then culture expanded.

In one embodiment, the mesenchymal lineage precursor or stem cells are culture expanded. In one embodiment the mesenchymal lineage precursor or stem cells are isolated, or isolated and enriched, and culture expanded *ex vivo or in vitro* prior to cryopreservation. In another example, the mesenchymal lineage precursor or stem cells are isolated, or isolated and enriched, cryopreserved, thawed and subsequently culture expanded. In yet another example, the mesenchymal lineage precursor or stem cells are culture expanded prior to and after cryopreservation.

In one embodiment, the mesenchymal lineage precursor or stem cells comprise at least 5% of the cell population of the composition.

In one embodiment, the composition is cryopreserved in 42.5% Profreeze^{™}/50% αMEM/7.5% DMSO.

In one embodiment, the composition is cryopreserved in Plasmalyte-A, 25%HSA and DMSO.

Although the scope of the present invention is not to be limited to any theoretical reasoning, the present inventors have found that mesenchymal lineage precursor or stem cells which inhibit the proliferation of T cells by at least about 65% are particularly useful in inhibiting immune responses, and more particularly such mesenchymal lineage precursor or stem cells are useful in the prevention or treatment of graft versus host disease; solid organ transplant rejection such as, for example, heart transplant rejection, liver transplant rejection, pancreas transplant rejection, intestine transplant rejection, and kidney transplant rejection; and autoimmune diseases such as, for example, rheumatoid arthritis, multiple sclerosis, Type I diabetes, Crohn's disease, Guillain-Barre syndrome, lupus erythematosus, myasthenia gravis, optic neuritis, psoriasis, Graves' disease, Hashimoto's disease, Ord's thyroiditis, aplastic anemia, Reiter's syndrome, autoimmune hepatitis, primary biliary cirrhosis, antiphospholipid antibody syndrome, opsoclonus myoclonus syndrome, temporal arteritis, acute disseminated encephalomyelitis, Goodpasture's syndrome, Wegener's granulomatosis, coeliac disease, pemphigus, polyarthritis, warm autoimmune hemolytic anemia, and scleroderma.

The present inventors have also found that the mesenchymal lineage precursor or stem cells are also useful for the treatment of inflammatory diseases, in particular T-cell mediated-inflammatory disorders.

The present invention also provides a method for producing a population of mesenchymal lineage precursor or stem cells which comprises one or more of the following steps:
culturing a population of mesenchymal lineage precursor or stem cells in a culture medium comprising recombinant trypsin;
culturing the cells in a cell factory with one or more attached air filters;
concentrating and/or washing the cells with tangential flow filtration (TFF); or
passing the harvested cells through a dual screen mesh filter, thereby reducing visible particulates and/or cell aggregates.

In one embodiment, the method comprises
culturing a population of mesenchymal lineage precursor or stem cells in a culture medium comprising recombinant trypsin; and
concentrating and/or washing the cells with tangential flow filtration (TFF).

In one embodiment, the method comprises
culturing a population of mesenchymal lineage precursor or stem cells in a culture medium comprising recombinant trypsin;
concentrating and/or washing the cells with tangential flow filtration (TFF); and
passing the harvested cells through a dual screen mesh filter, thereby reducing visible particulates and/or cell aggregates.

In one embodiment the method comprises the following steps:
culturing a population of mesenchymal lineage precursor or stem cells in a culture medium comprising recombinant trypsin;
culturing the cells in a cell factory with one or more attached air filters;
concentrating and/or washing the cells with tangential flow filtration (TFF); and
passing the harvested cells through a dual screen mesh filter, thereby reducing visible particulates and/or cell aggregates.

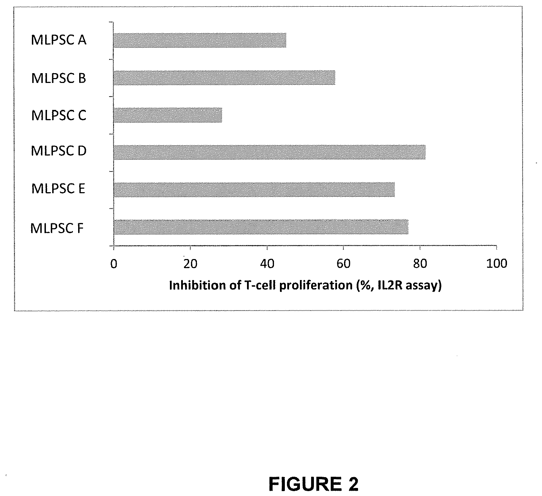

In one embodiment, the method comprises one or more or all of the steps outlined in Figure 2.

In one embodiment, the mesenchymal lineage precursor or stem cells are isolated by immunoselection. For example, the mesenchymal lineage precursor or stem cells isolated by immunoselection may be STRO-1+ mesenchymal precursor cells or progeny thereof.

In one embodiment, the mesenchymal lineage precursor or stem cells are isolated by plastic adherence technology. For example, the mesenchymal lineage precursor or stem cells isolated by plastic adherence technology may be mesenchymal stem cells or progeny thereof.

The present inventors have also developed a potency assay to measure the biological activity or therapeutic efficacy of cellular therapy products comprising mesenchymal lineage precursor or stem cells.

Accordingly, the present disclosure also provides a method for determining the potency of mesenchymal lineage precursor or stem cells comprising:
(i) obtaining a population of cells comprising mesenchymal lineage precursor or stem cells, wherein the cells have been cryopreserved and thawed;
(ii) co-culturing the cells in a culture medium with a population of cells comprising T cells;
(iii) determining the level of inhibition of T cell IL-2Rα expression, wherein an amount of ≥65% inhibition is indicative of biological activity or therapeutic efficacy of the mesenchymal lineage precursor or stem cells. For example, an amount of at least about 70% inhibition, at least about 75% inhibition, at least about 80% inhibition, at least about 85% inhibition, or at least about 90% inhibition is indicative of biological activity of therapeutic efficacy.

In one embodiment, an amount of at least about 65% inhibition is indicative of the cells therapeutic efficacy in inhibiting immune responses.

In one or a further embodiment, an amount of at least about 65% inhibition is indicative of the cells therapeutic efficacy in preventing or treating graft versus host disease.

The present disclosure also provides a method for selecting potent mesenchymal lineage precursor or stem cells comprising:
(i) obtaining a population of cells comprising mesenchymal lineage precursor or stem cells, wherein the cells have been cryopreserved and thawed;
(ii) co-culturing the cells in a culture medium with a population of cells comprising T cells;
(iii) selecting cells which exhibit a level of inhibition of T cell IL-2Rα expression, of ≥65% inhibition.

In one embodiment, the assay method or selection method described above is used to determine the potency of an enriched population of mesenchymal lineage precursor stem cells. For example, the mesenchymal lineage precursor or stem cells are enriched for mesenchymal stem cells. In another embodiment, the mesenchymal lineage precursor or stem cells are enriched by selection of STRO-1+ cells. In one embodiment, the mesenchymal lineage precursor cells are STRO-1^{bright} cells.

In one or a further embodiment, the assay or selection method is used to determine the potency of or select an *ex vivo* or *an in vitro* expanded population of mesenchymal lineage precursor or stem cells. In one example, the mesenchymal lineage precursor or stem cells are isolated, or isolated and enriched, and culture expanded *ex vivo or in vitro* prior to cryopreservation. In another example, the mesenchymal lineage precursor or stem cells are isolated, or isolated and enriched, cryopreserved, thawed and subsequently culture expanded. In yet another example, the mesenchymal lineage precursor or stem cells are culture expanded prior to and after cryopreservation.

In one embodiment, the mesenchymal lineage precursor or stem cells are human mesenchymal lineage precursor or stem cells.

In one embodiment, the T cells are human T cells. In another embodiment, the T cells express CD4 and CD8. In another embodiment, the T cells express CD69 and/or CD137.

In one or a further example, the population comprising T cells is a population of peripheral blood mononuclear cells (PBMCs).

In one embodiment, the assay or selection method comprises culturing the mesenchymal lineage precursor or stem cells with T cells in a culture medium comprising one or more T cell stimulatory ligands. For example, the culture medium comprises an anti-CD3 antibody or a fragment thereof and an anti-CD28 antibody or a fragment thereof. In another or further embodiment, the method comprises culturing the mesenchymal lineage precursor or stem cells with T cells that have been stimulated and/or activated prior to co-culture with the mesenchymal lineage precursor cells.

In one embodiment, the assay or selection method comprises culturing the cells in DMEM supplemented with 10% FBS and 2mM glutamine and optionally, comprising one or more T cell stimulatory ligands.

In one embodiment, the method comprises co-culturing mesenchymal lineage precursor or stem cells and T cells at a ratio of about 1 mesenchymal lineage precursor or stem cell:2 T cells, or less. For example, 1:3, 1:4, 1:5, 1:10, 1:20, 1:30, 1:40, 1:50, 1:60, 1:70 1:80, 1:90, or 1 mesenchymal lineage precursor or stem cell: 100 T cells, or less.

In one embodiment, the method comprises co-culturing the cells for 60 to 84 hours prior to determining IL-2Rα expression.

In one or a further embodiment, the method comprises collecting the cells following co-culture and lysing them to produce a cell lysate.

In one or a further embodiment, the method comprises determining the amount of IL-2Rα in the cell lysate by enzyme-linked immunosorbent assay (ELISA).

In one example, the ELISA comprises:
(i) adding sample diluent to each well of a microplate precoated with a monoclonal antibody specific for IL-2Rα;
(ii) adding a co-cultured sample to a well of a microplate precoated with a monoclonal antibody specific for IL-2Rα;
(iii) incubating the microplate for sufficient time to allow for the monoclonal antibody specific for IL-2Rα to specifically bind to any IL-2Rα in the sample;
(iv) washing the microplate;
(v) adding IL-2Rα conjugate to the well;
(vi) incubating the microplate for sufficient time to allow the conjugate to specifically bind to any captured IL-2Rα;
(vii) washing the microplate;
(viii) adding a substrate solution to the well;
(ix) incubating the microplate for sufficient time for colour development;
(x) adding a stop solution to the well;
(xi) reading optical density on a microplate reader set to 450 nm with wavelength correction at 570 nm;
(xii) determining the concentration of IL-2Rα.

In one embodiment, the method further comprises:
preparing serial dilutions of a IL-2Rα standard in a sample diluent to give, for example, final concentrations ranging from 7.8 to 500 pg/ml;
adding the standards to the microplate before step (iii);
constructing a standard curve using a four parameter logistic curve fit; and
determining the concentration of IL-2Rα by reference to the standard curve.

In one embodiment, the method further comprises:
determining TNFR1 expression of the population of mesenchymal lineage precursor or stem cells, wherein an amount of ≥ 100 pg/ml TNFR1 is indicative of biological activity or therapeutic efficacy of the mesenchymal lineage precursor or stem cells. For example, an amount of at least about 101 pg/ml TNFR1, at least about 102 pg/ml TNFβ1, at least about 103 pg/ml TNFR1, at least about 104 pg/ml TNFR1, at least about 105 pg/ml TNFR, at least about 106 pg/ml TNFR1, at least about 107 pg/ml TNFR1, at least about 108 pg/ml TNFR1, at least about 109 pg/ml TNFR1, or at least about 110 pg/ml TNFR1, or at least about 150 pg/ml, or at least about 200 pg/ml, or at least about 250 pg/ ml, or at least about 300 pg/ml, or at least about 320 pg/ml, or at least about 330 pg/ml, or at least about 340 pg/ml, or at least about 350 pg/ml, is indicative of biological activity or therapeutic efficacy.

The present disclosure also provides a method of inhibiting an immune response in a subject in need thereof, the method comprising administering a composition comprising the population of MLPSCs of the disclosure to the subject.

The present disclosure also provides a method of preventing or treating an inflammatory disorder in a subject in need thereof, the method comprising administering a composition comprising MLPSCs of the present disclosure to the subject. The inflammatory disorder may be a a T-cell mediated inflammatory disorder.

The present disclosure also provides a method of preventing, alleviating the development of, or treating graft versus host disease in a subject, comprising administering to the subject the composition comprising MLPSCs of the present disclosure to the subject.

The present disclosure also provides a method of preventing graft versus host disease in a subject, the method comprising administering hematopoietic stem cells co-cultured with MLPSCs of the present disclosure to the subject.

In one embodiment, the composition is administered to the subject at a dose of less than 3 × 10⁶ cells/kg body weight once per week (qw).

The present disclosure also provides a method for preventing, alleviating the development of, or treating graft versus host disease (GVHD) in a mammalian subject, comprising administering to the subject, MLPSCs and/or progeny cells thereof at a dose of less than 3 × 10⁶ cells/kg body weight once per week (qw).

In one embodiment, the subject is administered MLPSCs at a dose of about 2 × 10⁶ cells/kg body weight qw. In another embodiment, the subject is administered cells at a dose up to 2 × 10⁶ cells/kg body weight qw. In another embodiment, the subject is administered a maximal dose of 2 × 10⁶ cells/kg body weight qw. For the avoidance of doubt the terms "one week", "weekly" or "qw" is intended to mean a period once every 7 days.

In another embodiment, the MLPSCs are administered as a single dose once per week (qw). In another embodiment, the MLPSCs are administered as a divided dose per week (i.e. 7 days). For example, the subject may receive two doses with each dose of 1 × 10⁶ cells/kg body weight over the course of one week. In other embodiments, the subject may receive two or more doses per week wherein the total dose received is 2 × 10⁶ cells/kg body weight.

In one embodiment the graft comprises allogenic cells. In another embodiment, the graft comprises autologous cells.

In one embodiment, the MLPSCs administered to the subject are MPCs and/or progeny cells thereof.

In another embodiment, the MLPSCs administered to the subject are MSCs and/or progeny cells thereof.

In one embodiment, the MLPSCs and/or progeny cells thereof are delivered as a single dose qw. In another embodiment, the MLPSCs and/or progeny cells thereof are delivered as a divided dose over the course of one week.

In one embodiment, the mammalian subject is a human subject. In one embodiment, the subject is a pediatric subject. In another embodiment, the subject is an adult subject.

In another embodiment, the subject according to the present disclosure is one having a malignant or genetic disorder of the blood (e.g. cancer). In a further example, the subject has received, is receiving or is about to receive a donor graft comprising hematopoietic cells.

The graft comprising hematopoietic cells may be selected from the group consisting of blood, peripheral blood mononuclear cells (PBMCs), blood products, or solid organs in which hematopoietic cells are present. In one example, the graft comprises hematopoietic stem cells (HSCs).

In one embodiment, the subject has acute GVHD. Symptoms of acute GVHD are typically graded by standard clinical criteria (Glucksberg H. et al. (1974) Transplantation 1974;18(4):295-304).

In one embodiment, the subject has received steroid therapy prior to administration of the mesenchymal lineage precursor or stem cells. In one example, the steroid is methylprednisolone. In another example, the steroid is administered to the subject at least three (3) days prior to administration of the MLPSCs and/or progeny cells thereof.

In one embodiment, the subject is administered MLPSCs and/or progeny cells thereof on the same day as receiving the graft (e.g. bone marrow or PBMCs).

The MLPSCs and/or progeny cells thereof may be administered to the subject at an appropriate time which may be during or following transplantation of the graft. For example, for prophylactic purposes the MLPSCs and/or progeny cells thereof may be administered to the subject beginning on the day of transplantation of the graft. In another example, the MLPSCs and/or progeny cells thereof may be administered to the subject prior to receiving the graft. In another example, the MLPSCs and/or progeny cells thereof may be administered within 7 days, within 5 days, within 3 days, or within 2 days prior to receiving the graft. In another example, the MLPSCs and/or progeny cells thereof are administered to the subject the day before receiving the graft.

In another embodiment, the MLPSCs and/or progeny cells thereof are administered to the subject after the subject has been determined to be steroid refractory. While there is no generally agreed upon definition of steroid-refractory acute GVHD, typically steroid refractory acute GVHD refers to GVHD that worsens after 3-5 days of steroid treatment, that does not improve after 5-7 days or that fails to remit completely after 14 days. In another example, the MLPSCs and/or progeny cells thereof are administered to the subject after at least three days of steroid or immunosuppressive treatment. In another example, the MLPSCs and/or progeny cells thereof are administered to the subject after at least one month of steroid or immunosuppressive treatment. In one example, a steroid refractory subject is one that has failed to respond to steroid treatment for grades B-D acute GVHD after at least three days of steroid (e.g. methylprednisolone or equivalent). In a further example, the subject has failed to respond to >1mg/kg/day of methylprednisolone or equivalent.

In another embodiment, the subject has received non-steroid immunosuppressive therapy prior to administration of MLPSCs and/or progeny cells thereof. In another example, the subject has received one or more non-steroid therapies selected from the group consisting of extracorporeal photophoresis (ECP), infliximab, ruxolitinib, mycophenolate mofetil (MMF), etanercept and basiliximab. In one example, MLPSCs and/or progeny cells thereof are administered to a subject who is refractory to immunosuppressive treatment with non-steroidal agents.

In one example, the subject is administered MLPSCs and/or progeny cells thereof until improvement in GVHD is observed. In another example, the subject is administered MLPSCs and/or progeny cells thereof until remission of GVHD is observed.

In one example, the administration of MLPSCs and/or progeny cells thereof prevents, alleviates or treats an adverse event selected from one or more of infusion-related reaction, hypertension, vomiting, nausea, brachycardia and fever. In one example, the administration of MLPSCs and/or progeny cells thereof reduces the number of adverse events experienced by the subject compared to a subject who has not received MLPSCs and/or progeny cells thereof.

In one example, the subject has acute GVHD Grade B, C or D. In another example, the GVHD involves the skin, gastrointestinal tract or liver or a combination of any one or more of these tissues.

In one example, the GVHD is a result of a T cell immune response. In one example, the T cells are from a donor and the antigen is from the recipient. For example, the T cells may be present in a transplant. In another embodiment, the T cells are from the recipient and the antigen is from the donor.

In another embodiment of this method, the mesenchymal lineage precursor or stem cells are genetically engineered to express a molecule to block co-stimulation of T-cells.

In another embodiment of this method, the mesenchymal lineage precursor or stem cells have been expanded in culture prior to administration to the subject.

In one embodiment, the MLPSCs and/or progeny cells thereof are administered in the form of a pharmaceutically acceptable composition. In a further example, the pharmaceutically acceptable composition comprises a pharmaceutically acceptable carrier and/or excipient.

The MLPSCs and/or progeny cells thereof may be administered weekly to the subject for each of four (4) consecutive weeks. In another example, MLPSCs and/or progeny cells thereof are administered to the subject weekly for each of eight consecutive weeks. In another example, the subject's GVHD is assessed after the four once-weekly infusions and if the subject's GVHD response is partial or mixed, the subject is eligible to receive an additional four once-weekly infusions. In one example, the subject is administered up to a maximum of eight MLPSCs and/or progeny cells thereof. In another example, the subject is administered a total of eight MPCs and/or progeny cells thereof. In another example, the subject is administered MLPSCs and/or progeny cells thereof infusions on a weekly basis until at least a partial or complete response is observed.

In one embodiment, the GVHD status or grading in the subject is assessed at baseline (screening), day 0.

In another embodiment, the GVHD status or grading in the subject is assessed at Day 14, Day 28, Day 56 and Day 100. In another embodiment, GVHD is assessed at some of these days, for example, baseline (Day 0), Day 28 and Day 100.

The subject may be assessed as having a complete response, partial response, mixed response, worsening response or no response.

The present disclosure also provides a composition comprising MLPSCs and/or progeny cells thereof at a dose of less than 3 × 10⁶ cells/kg body weight on a weekly basis for use in preventing, alleviating the development of, or treating graft versus host disease (GVHD) in a mammalian subject.

In one embodiment, the composition comprises MLPSCs and/or progeny cells thereof at a dose of about 2 × 10⁶ cells/kg body weight. In another embodiment, the composition comprises MPCs and/or progeny cells thereof at a dose up to 2 × 10⁶cells/kg body weight. In another embodiment the composition comprises a maximal dose of 2 × 10⁶ cells/kg body weight thereof.

In one example, the composition is a pharmaceutical composition.

The present disclosure also provides use of MLPSCs and/or progeny cells thereof at a dose of less than 3 × 10⁶ cells/kg body weight in the manufacture of a medicament for preventing development of, or treating GVHD in a mammalian subject. In one example, the MLPSCs and/or progeny cells thereof are intended for administration to a subject in need thereof once per week (qw).

In one example, the medicament comprises MLPSCs and/or progeny cells thereof at a dose of about 2 × 10⁶ cells/kg body weight. In another example, the medicament comprises MLPSCs and/or progeny cells thereof at a dose up to 2 × 10⁶ cells/kg body weight. In another example, the medicament comprises a maximal dose of 2 × 10⁶ cells/kg body weight.

In one example, the mesenchymal lineage precursor or stem cells is a population of cells enriched for STRO-1^{bright} cells. In another example, the mesenchymal lineage precursor or stem cells is a population of cells enriched for one or more additional markers selected from TNAP+, VCAM-1+, THY-1+, STRO-2+, STRO-4+ (HSP-90β) and/or CD146+.

In one example, the mesenchymal lineage precursor or stem cells is a population of mesenchymal stem cells

In one example, the MLPSCs and/or progeny cells thereof are administered systemically. For example, the MLPSCs and/or progeny cells thereof may be administered intravenously, intra-arterially, intramuscularly, subcutaneously, into an aorta, into an atrium or ventricle of the heart or into a blood vessel connected to an organ, e.g., an abdominal aorta, a superior mesenteric artery, a pancreatic duodenal artery or a splenic artery.

In another embodiment, the methods of the disclosure further comprise administering an immunosuppressive agent. The immunosuppressive agent may be administered for a time sufficient to permit the transplanted hematopoietic cells to be functional. The immunosuppressive agent may be selected from one or more of the following, including but not limited to corticosteroids such as prednisone, budesonide and prednisolone; calcineurin inhibitors such as cyclosporine and tacrolimus; mTOR inhibitors such as sirolimus and everolimus; IMDH inhibitors such as azathioprine, leflunomide and mycophenolate; a biologic such as abatacept, adalimumab, etanercept, infliximab or rituximab.

In one example, the immunosuppressive agent is cyclosporine. The cyclosporine may be administered at a dosage of from 5 to 40 mg/kg body wt.

### Brief Description of Drawings

**Figure 1****:** Morphology of unstimulated human PBMC, stimulated human PBMC and co-cultured human MPC (long, flat) and human PBMC (round, circular and some aggregates).
**Figure 2****:** Results of the T cell proliferation assay (% inhibition of IL2R) performed on performed on three different samples of MLPSCs produced under the previous manufacturing conditions (i.e. samples MLPSC A, MLPSC B and MLPSC C) and three different samples of improved immunoselected MLPSCs with (i.e. samples MLPSC D, MLPSC E and MLPSC F).
**Figure 3****:** Results of assays for TNFR1 expression performed on three different samples of MLPSCs produced under the previous manufacturing conditions (i.e. samples MLPSC A, MLPSC B and MLPSC C) and three different samples of improved immunoselected MLPSCs with (i.e. samples MLPSC D, MLPSC E and MLPSC F).
**Figure 4****:** shows survival through to 100 Days following infusion of MPCs for responder versus non-responder subjects. All nine subjects who responded at Day 28 survived to Day 100, however only one in three non-responders at Day 28 survived to Day 100 (p value =0.0068).
**Figure 5****:** Steps involved in the improved culture expanded MLPSC manufacturing process.
**Figure 6****:** Results of assays for TNFR1 expression performed on 10 different MLPSC lot products produced under improved manufacturing conditions.
**Figure 7****:** Results of T cell proliferation assay (% inhibition of IL2R) performed on 10 different MLPSC lot products produced under improved manufacturing conditions.

### Description of Embodiments

### General techniques and definitions

Throughout this specification, unless specifically stated otherwise or the context requires otherwise, reference to a single step, composition of matter, group of steps or group of compositions of matter shall be taken to encompass one and a plurality (i.e., one or more) of those steps, compositions of matter, group of steps or group of compositions of matter.

Those skilled in the art will appreciate that the disclosure described herein is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features.

The present disclosure is not to be limited in scope by the specific embodiments described herein, which are intended for the purpose of exemplification only. Functionally-equivalent products, compositions and methods are clearly within the scope of the disclosure.

Any example disclosed herein shall be taken to apply *mutatis mutandis* to any other example unless specifically stated otherwise.

Unless specifically defined otherwise, all technical and scientific terms used herein shall be taken to have the same meaning as commonly understood by one of ordinary skill in the art (e.g., in cell culture, molecular genetics, stem cell differentiation, immunology, immunohistochemistry, protein chemistry, and biochemistry).

Unless otherwise indicated, the stem cells, cell culture, and surgical techniques utilized in the present disclosure are standard procedures, well known to those skilled in the art. Such techniques are described and explained throughout the literature in sources such as Perbal, 1984; Sambrook & Green, 2012; Brown, 1991; Glover & Hames, 1995 and 1996; Ausubel., 1987 including all updates untill present; Harlow & Lane, 1988; and Coligan et al., 1991 including all updates until present.

As used in this specification and the appended claims, terms in the singular and the singular forms "a," "an" and "the," for example, optionally include plural referents unless the content clearly dictates otherwise.

The term "graft versus host disease" or "GVHD" refers to a complication of allogeneic hematopoietic cell transplantation in which the tissues of the host, most frequently the skin, liver and intestine are damaged by lymphocyte from the donor. This disease is discussed in more detail below.

The term "subject" as used herein refers to a mammal including human and non-human animals. More particularly, the mammal is a human. Terms such as "subject", "patient" or "individual" are terms that can, in context, be used interchangeably in the present disclosure. In certain examples, the subject may be an adult or a child (pediatric) subject.

An "effective amount" refers to at least an amount effective, at dosages and for periods of time necessary, to achieve the desired therapeutic or prophylactic result. An effective amount can be provided in one or more administrations. In some examples of the present disclosure, the term "effective amount" is used to refer to an amount necessary to effect treatment of a disease or condition as hereinbefore described. The effective amount may vary according to the disease or condition to be treated and also according to the weight, age, racial background, sex, health and/or physical condition and other factors relevant to the mammal being treated. Typically, the effective amount will fall within a relatively broad range (e.g. a "dosage" range) that can be determined through routine trial and experimentation by a medical practitioner. The effective amount can be administered in a single dose or in a dose repeated once or several times over a treatment period.

A "therapeutically effective amount" is at least the minimum concentration required to effect a measurable improvement of a particular disorder (e.g. GVHD). A therapeutically effective amount herein may vary according to factors such as the disease state, age, sex, and weight of the patient, and the ability of the cellular composition to elicit a desired response in the individual. A therapeutically effective amount is also one in which any toxic or detrimental effects of the composition are outweighed by the therapeutically beneficial effects. In the case of GVHD, a therapeutically effective amount can reduce the severity, inhibit or delay progression of GVHD and/or relieve to some extent one or more of the symptoms associated with the disorder.

The term "preventing" or "prevent" as used herein means preventing, delaying and/or reducing the severity of the symptoms associated with GVHD. This is distinguished from "treatment" which occurs following the onset of the first symptoms of GVHD.

The term "alleviating" as used herein refers to a reduction in the severity of the disease and/or one or more symptoms associated with the disease (e.g. GVHD). It does not imply complete abrogation or elimination of disease.

As used herein, the term "treatment" refers to clinical intervention designed to alter the natural course of the individual or cell being treated during the course of clinical pathology. Desirable effects of treatment include decreasing the rate of disease progression, ameliorating or palliating the disease state, and remission or improved prognosis. An individual is successfully "treated", for example, if one or more symptoms associated with a disease are mitigated or eliminated.

The term "complete response" or "CR" as referred to herein is defined as the complete resolution of acute GVHD symptoms in all organs, without secondary GVHD therapy.

The term "partial response" or "PR" as referred to herein is defined as improvement by at least one GVHD stage in all initial GVHD target organs without complete resolution and without worsening in any other GVHD target organs, without secondary GVHD therapy.

The term "no response" or "NR" as referred to herein is defined as the same grade of GVHD or progression of GVHD in any organ (e.g. deterioration in at least one evaluable organ symptom by one stage or more) or death, or the addition of secondary GVHD therapy.

The term "worsening" as used herein refers to worsening of GVHD progression in at least one organ with or without amelioration in any organ.

The term "very good partial response (VGPR)" refers to fulfilment of the complete response criteria with the exception of one or more of (i) non-progressive stage 1 rash, not including residual faint erythema or hyperpigmentation, (ii) resolving elevation of total serum bilirubin <25% of baseline; or (iii) minimal gastrointestinal symptoms.

The term "mixed response" or "MR" as used herein refers to improvement in at least one evaluable organ stage with worsening in another.

The term "progression" refers to deterioration in at least one organ system by one stage or more with no improvement in any other organ.

The term "adult" as used herein means a human subject of 18 years of age and older.

The term "pediatric" as used herein means a human subject ranging in age from birth up to and including 17 years of age.

The term "acute GVHD" as used herein refers to GVHD that usually occurs within the first 6 months of receiving a graft, e.g. bone marrow transplant. It can occur within a matter of days of receiving a graft.

The term "chronic GVHD" as used herein refers to GVHD that usually commences more than 3 months after receiving a graft. Symptoms of chronic GVHD can last a lifetime.

The term "graft" as used herein refers to a biological sample selected from bone marrow, blood (e.g. whole blood or peripheral blood mononuclear cells (PBMCs), blood products, or solid organs in which hematopoietic cells are present.

The term "allogeneic" as used herein refers to a graft (e.g. hematopoietic cells) which are donated by an individual whose genetic characteristics differ from those of the recipient, especially in regards to the major histocompatibility complex (MHC) and minor histocompatibility agents expressed on the surface of the individual's cells.

The term "autologous" as used herein refers to a graft (e.g. hematopoietic cells present in the bone marrow or peripheral blood) that uses the subject's own cells. The cells are usually harvested in advance of the subject undergoing treatment (e.g. with chemotherapy), stored and then re-infused back into the subject.

The term "steroid refractory" as used herein refers to GVHD that worsens after 3-5 days of steroid treatment that does not improve after 5-7 days or that fails to remit completely after 14 days.

The term "and/or", e.g., "X and/or Y" shall be understood to mean either "X and Y" or "X or Y" and shall be taken to provide explicit support for both meanings or for either meaning.

As used herein, the term about, unless stated to the contrary, refers to +/- 10%, more preferably +/- 5%, of the designated value.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### Mesenchymal lineage precursor or stem cells

As used herein, the term "mesenchymal lineage precursor or stem cells" refers to undifferentiated multipotent cells that have the capacity to self-renew while maintaining multipotency and the capacity to differentiate into a number of cell types either of mesenchymal origin, for example, osteoblasts, chondrocytes, adipocytes, stromal cells, fibroblasts and tendons, or non-mesodermal origin, for example, hepatocytes, neural cells and epithelial cells..

The term "mesenchymal lineage precursor or stem cells" includes both parent cells and their undifferentiated progeny. The term also includes mesenchymal precursor cells (MPC), multipotent stromal cells, mesenchymal stem cells, perivascular mesenchymal precursor cells, and their undifferentiated progeny.

Mesenchymal lineage precursor or stem cells can be autologous, allogeneic, xenogeneic, syngeneic or isogeneic. Autologous cells are isolated from the same individual to which they will be reimplanted. Allogeneic cells are isolated from a donor of the same species. Xenogeneic cells are isolated from a donor of another species. Syngeneic or isogeneic cells are isolated from genetically identical organisms, such as twins, clones, or highly inbred research animal models.

Mesenchymal lineage precursor or stem cells reside primarily in the bone marrow, but have also been shown to be present in diverse host tissues including, for example, cord blood and umbilical cord, adult peripheral blood, adipose tissue, trabecular bone and dental pulp.

Mesenchymal lineage precursor or stem cells can be isolated from host tissues and enriched for by immunoselection. For example, a bone marrow aspirate from a subject may be further treated with an antibody to STRO-1 or TNAP to enable selection of mesenchymal lineage precursor or stem cells. In one example, the mesenchymal lineage precursor or stem cells can be enriched for by using the STRO-1 antibody described in Simmons & Torok-Storb, 1991.

STRO-1+ cells are cells found in bone marrow, blood, dental pulp cells, adipose tissue, skin, spleen, pancreas, brain, kidney, liver, heart, retina, brain, hair follicles, intestine, lung, lymph node, thymus, bone, ligament, tendon, skeletal muscle, dermis, and periosteum; and are capable of differentiating into germ lines such as mesoderm and/or endoderm and/or ectoderm. Thus, STRO-1+ cells are capable of differentiating into a large number of cell types including, but not limited to, adipose, osseous, cartilaginous, elastic, muscular, and fibrous connective tissues. The specific lineage-commitment and differentiation pathway which these cells enter depends upon various influences from mechanical influences and/or endogenous bioactive factors, such as growth factors, cytokines, and/or local microenvironmental conditions established by host tissues.

The term "enriched" as used herein describes a population of cells in which the proportion of one particular cell type or the proportion of a number of particular cell types is increased when compared with an untreated population of the cells (e.g., cells in their native environment). In one example, a population enriched for STRO-1+ cells comprises at least about 0.1% or 0.5% or 1% or 2% or 5% or 10% or 15% or 20% or 25% or 30% or 50% or 75% STRO-1+ cells. In this regard, the term "population of cells enriched for STRO-1+ cells" will be taken to provide explicit support for the term "population of cells comprising X% STRO-1+ cells", wherein X% is a percentage as recited herein. The STRO-1+ cells can, in some examples, form clonogenic colonies, for example, CFU-F (fibroblasts) or a subset thereof (e.g., 50% or 60% or 70% or 70% or 90% or 95%) can have this activity.

In one example, the population of cells is enriched from a cell preparation comprising STRO-1+ cells in a selectable form. In this regard, the term "selectable form" will be understood to mean that the cells express a marker (e.g., a cell surface marker) permitting selection of the STRO-1+ cells. The marker can be STRO-1, but need not be. For example, as described and/or exemplified herein, cells (e.g., MPCs) expressing STRO-2 and/or STRO-3 (TNAP) and/or STRO-4 and/or VCAM-1 and/or CD146 and/or 3G5 also express STRO-1 (and can be STRO-1^{bright}). Accordingly, an indication that cells are STRO-1+ does not mean that the cells are selected by STRO-1 expression. In one example, the cells are selected based on at least STRO-3 expression, e.g., they are STRO-3+ (TNAP+).

Reference to selection of a cell or population thereof does not necessarily require selection from a specific tissue source. As described herein, STRO-1+ cells can be selected from or isolated from or enriched from a large variety of sources. That said, in some examples, these terms provide support for selection from any tissue comprising STRO-1+ cells or vascularized tissue or tissue comprising pericytes (e.g., STRO-1+ pericytes) or any one or more of the tissues recited herein.

In one example, the mesenchymal lineage precursor or stem cells of the disclosure express one or more markers individually or collectively selected from the group consisting of TNAP+, VCAM-1+, THY-1+, STRO-2+, STRO-4+ (HSP-90β), CD45+, CD146+, 3G5+.

By "individually" is meant that the disclosure encompasses the recited markers or groups of markers separately, and that, notwithstanding that individual markers or groups of markers may not be separately listed herein, the accompanying claims may define such marker or groups of markers separately and divisibly from each other.

By "collectively" is meant that the disclosure encompasses any number or combination of the recited markers or groups of markers, and that, notwithstanding that such numbers or combinations of markers or groups of markers may not be specifically listed herein, the accompanying claims may define such combinations or sub- combinations separately and divisibly from any other combination of markers or groups of markers.

A cell that is referred to as being "positive" for a given marker may express either a low (lo or dim or dull), intermediate (median) or a high (bright, bri) level of that marker depending on the degree to which the marker is present on the cell surface, where the terms relate to intensity of fluorescence or other marker used in the sorting process of the cells or flow cytometric analysis of the cells. The distinction of low (lo or dim or dull), intermediate (median), or high (bright, bri) will be understood in the context of the marker used on a particular cell population being sorted or analysed. A cell that is referred to as being "negative" for a given marker is not necessarily completely absent from that cell. This term means that the marker is expressed at a relatively very low level by that cell, and that it generates a very low signal when detectably labeled or is undetectable above background levels, for example, levels detected using an isotype control antibody.

The term "bright" or bri as used herein, refers to a marker on a cell surface that generates a relatively high signal when detectably labeled. Whilst not wishing to be limited by theory, it is proposed that "bright" cells express more of the target marker protein (for example, the antigen recognized by a STRO-1 antibody) than other cells in the sample. For instance, STRO-1^{bri} cells produce a greater fluorescent signal, when labeled with a FITC-conjugated STRO-1 antibody as determined by fluorescence activated cell sorting (FACS) analysis, than non-bright cells (STRO-1^{lo/dim/dull/intermediate/median}). In one example, the mesenchymal lineage precursor or stem cells are isolated from bone marrow and enriched for by selection of STRO-1+ cells. In this example, "bright" cells constitute at least about 0.1% of the most brightly labeled bone marrow mononuclear cells contained in the starting sample. In other examples, "bright" cells constitute at least about 0.1%, at least about 0.5%, at least about 1%, at least about 1.5%, or at least about 2%, of the most brightly labeled bone marrow mononuclear cells contained in the starting sample. In an example, STRO-1^{bright} cells have 2 log magnitude higher expression of STRO-1 surface expression relative to "background", namely cells that are STRO-1-. By comparison, STRO-1^{lo/dim/dull} and/or STRO-1^{intermediate/median} cells have less than 2 log magnitude higher expression of STRO-1 surface expression, typically about 1 log or less than "background".

In one example, the STRO-1+ cells are STRO-1^{bright}. In one example, the STRO-1^{bright} cells are preferentially enriched relative to STRO-1^{lo/dim/dull} or STRO-1^{intermediate/median} cells.

In one example, the STRO-1^{bright} cells are additionally one or more of TNAP+, VCAM-1+, THY-1+, STRO-2+, STRO-4+ (HSP-90β) and/or CD146+. For example, the cells are selected for one or more of the foregoing markers and/or shown to express one or more of the foregoing markers. In this regard, a cell shown to express a marker need not be specifically tested, rather previously enriched or isolated cells can be tested and subsequently used, isolated or enriched cells can be reasonably assumed to also express the same marker.

In one example, the STRO-1^{bright} cells are perivascular mesenchymal precursor cells as defined in WO 2004/85630, characterized by the presence of the perivascular marker 3G5.

As used herein the term "TNAP" is intended to encompass all isoforms of tissue non-specific alkaline phosphatase. For example, the term encompasses the liver isoform (LAP), the bone isoform (BAP) and the kidney isoform (KAP). In one example, the TNAP is BAP. In one example, TNAP refers to a molecule which can bind the STRO-3 antibody produced by the hybridoma cell line deposited with ATCC on 19 December 2005 under the provisions of the Budapest Treaty under deposit accession number PTA-7282.

Furthermore, in one example, the STRO-1+ cells are capable of giving rise to clonogenic CFU-F.

In one example, a significant proportion of the STRO-1+ cells are capable of differentiation into at least two different germ lines. Non-limiting examples of the lineages to which the cells may be committed include bone precursor cells; hepatocyte progenitors, which are multipotent for bile duct epithelial cells and hepatocytes; neural restricted cells, which can generate glial cell precursors that progress to oligodendrocytes and astrocytes; neuronal precursors that progress to neurons; precursors for cardiac muscle and cardiomyocytes, glucose-responsive insulin secreting pancreatic beta cell lines. Other lineages include, but are not limited to, odontoblasts, dentin-producing cells and chondrocytes, and precursor cells of the following: retinal pigment epithelial cells, fibroblasts, skin cells such as keratinocytes, dendritic cells, hair follicle cells, renal duct epithelial cells, smooth and skeletal muscle cells, testicular progenitors, vascular endothelial cells, tendon, ligament, cartilage, adipocyte, fibroblast, marrow stroma, cardiac muscle, smooth muscle, skeletal muscle, pericyte, vascular, epithelial, glial, neuronal, astrocyte and oligodendrocyte cells.

In one example, the mesenchymal lineage precursor or stem cells are mesenchymal stem cells (MSCs). The MSCs may be a homogeneous composition or may be a mixed cell population enriched in MSCs. Homogeneous MSC compositions may be obtained by culturing adherent bone marrow or periosteal cells, and the MSCs may be identified by specific cell surface markers which are identified with unique monoclonal antibodies. A method for obtaining a cell population enriched in MSCs using plastic adherence technology is described, for example, in US patent 5486359. MSC prepared by conventional plastic adherence isolation relies on the non-specific plastic adherent properties of CFU-F. Alternative sources for MSCs include, but are not limited to, blood, skin, cord blood, muscle, fat, bone, and perichondrium.

The mesenchymal lineage precursor or stem cells may be cryopreserved prior to administration to a subject.

In a preferred embodiment of the invention, the mesenchymal lineage precursor or stem cells are obtained from a master cell bank derived from mesenchymal lineage precursor or stem cells enriched from the bone marrow of healthy volunteers. The use of mesenchymal lineage precursor or stem cells derived from such a source is particularly advantageous for subjects who do not have an appropriate family member available who can serve as the mesenchymal lineage precursor or stem cell donor, or are in need of immediate treatment and are at high risk of relapse, disease-related decline or death, during the time it takes to generate mesenchymal lineage precursor or stem cells.

The present inventors have shown that mesenchymal precursor cells of the disclosure have unexpectedly high potency in terms of their ability to inhibit T cell proliferation after cryopreservation and thawing. In contrast, prior publications teach that cryopreserved mesenchymal stem cells display impaired immunosuppressive properties following thawing (Francois et al., 2012; Chinnadurai et al., 2016).

The isolated or enriched mesenchymal lineage precursor or stem cells can be expanded *ex vivo* or *in vitro* by culture. As will be appreciated by those skilled in the art, the isolated or enriched mesenchymal lineage precursor or stem cells can be cryopreserved, thawed and subsequently or further expanded *ex vivo* or *in vitro* by culture.

The cultured mesenchymal lineage precursor or stem cells are phenotypically different to cells *in vivo.* For example, in one embodiment they express one or more of the following markers, CD44, NG2, DC146 and CD140b.

The cultured mesenchymal lineage precursor or stem cells are biologically different to cells *in vivo,* having a higher rate of proliferation compared to the largely non-cycling (quiescent) cells *in vivo.*

In one example, a population of cells enriched for mesenchymal lineage precursor or stem cells is seeded at about 6000 to 7000 viable cells/cm² in serum-supplemented culture medium, for example, Dulbecco's Modified Eagle medium (DMEM) supplemented with 10% fetal bovine serum (FBS) and 2mM glutamine, and allowed to adhere to the culture vessel overnight at 37°C, 20% O₂. In an embodiment, the cells are seeded at about 6000, 6100, 6200, 6300, 6400, 6500, 6600, 6700, 6800, 6810, 6820, 6830, 6840, 6850, 6860, 6870, 6880, 6890, 6890, 6900, 6910, 6920, 6930, 6940, 6970, 6980, 6990, or 7000 viable cells/cm², preferably at about 6850 to 6860 viable cells/cm². The culture medium is subsequently replaced and the cells cultured for a total of 68 to 72 hours at 37°C, 5% O₂ prior to co-culturing with T cells and determining the amount of IL-2Rα expressed by the T cells.

### Ang1 and VEGF levels

In an example, mesenchymal lineage precursor or stem cells express Ang1 in an amount of at least 0.1 µg/106 cells. However, in other examples, MLPSCs express Ang1 in an amount of at least 0.2 µg/106 cells, 0.3 µg/106 cells, 0.4 µg/10⁶ cells, 0.5 µg/10⁶ cells, 0.6 µg/10⁶ cells, 0.7 µg/10⁶ cells, 0.8 µg/10⁶ cells, 0.9 µg/10⁶ cells, 1 µg/10⁶ cells, 1.1 µg/10⁶ cells, 1.2 µg/10⁶ cells, 1.3 µg/10⁶ cells, 1.4 µg/10⁶ cells, 1.5 µg/10⁶ cells.

In another example, MLPSCs express VEGF in an amount less than about 0.05 µg/10⁶ cells. However, in other examples, mesenchymal precursor cells express VEGF in an amount less than about 0.05 µg/10⁶ cells, 0.04 µg/10⁶ cells, 0.03 µg/10⁶ cells, 0.02 µg/10⁶ cells, 0.01 µg/10⁶ cells, 0.009 µg/10⁶ cells, 0.008 µg/10⁶ cells, 0.007 µg/10⁶ cells, 0.006 µg/10⁶ cells, 0.005 µg/10⁶ cells, 0.004 µg/10⁶ cells, 0.003 µg/10⁶ cells, 0.002 µg/10⁶ cells, 0.001 µg/10⁶ cells.

The amount of cellular Ang1 and/or VEGF that is expressed in a composition or culture of MLPSCs may be determined by methods known to those skilled in the art. Such methods include, but are not limited to, quantitative assays such as quantitative ELISA assays, for example. In this example, a cell lysate from a culture of mesenchymal precursor cells is added to a well of an ELISA plate. The well may be coated with a primary antibody, either a monoclonal or a polyclonal antibody(ies), against the Ang1 or VEGF. The well then is washed, and then contacted with a secondary antibody, either a monoclonal or a polyclonal antibody(ies) , against the primary antibody. The secondary antibody is conjugated to an appropriate enzyme, such as horseradish peroxidase, for example. The well then may be incubated, and then is washed after the incubation period. The wells then are contacted with an appropriate substrate for the enzyme conjugated to the secondary antibody, such as one or more chromogens. Chromogens which may be employed include, but are not limited to, hydrogen peroxide and tetramethylbenzidine. After the substrate(s) is (are) added, the well is incubated for an appropriate period of time. Upon completion of the incubation, a "stop" solution is added to the well in order to stop the reaction of the enzyme with the substrate(s). The optical density (OD) of the sample is then measured. The optical density of the sample is correlated to the optical densities of samples containing known amounts of Ang1 or VEGF in order to determine the amount of Ang1 or VEGF expressed by the culture of stem cells being tested.

In another example, MLPSCs express Ang1:VEGF at a ratio of at least about 2:1. However, in other examples, mesenchymal precursor cells express Ang1:VEGF at a ratio of at least about 10:1, 15:1, 20:1, 21:1, 22:1, 23:1, 24:1, 25:1, 26:1, 27:1, 28:1, 29:1, 30:1, 31:1, 32:1, 33:1, 34:1, 35:1, 50:1.

Methods for determining the Ang1:VEGF expression ratio will be apparent to one of skill in the art. For example Ang1 and VEGF expression levels can be quantitated via quantitative ELISA as discussed above. After quantifying the levels of Ang1 and VEGF, a ratio based on the quantitated levels of Ang1 and VEGF could be represented as: (level of Ang1 / level of VEGF) = Ang1:VEGF ratio.

In an example, the MLPSCs of the present disclosure are not genetically modified to express Ang1 and/or VEGF at an above exemplified level or ratio. Cells that are not genetically modified to express Ang1 and/or VEGF have not been modified by transfection with a nucleic acid expressing or encoding Ang1 and/or VEGF. For the avoidance of doubt, in the context of the present disclosure a mesenchymal precursor cell transfected with a nucleic acid encoding Ang1 and/or VEGF would be considered genetically modified. In the context of the present disclosure cells not genetically modified to express Ang1 and/or VEGF naturally express Ang1 and/or VEGF to some extent without transfection with a nucleic acid encoding Ang1 and/or VEGF1.

### T cells

The potency assay of the disclosure requires co-culture of the mesenchymal lineage precursor or stem cells with T-cells. In an embodiment, the mesenchymal lineage precursor or stem cells are co-cultured with T-cells in a culture medium comprising at least one T-cell stimulatory ligand. In an embodiment or a further embodiment, the T cells are activated. The T cells may be first stimulated or activated prior to co-culture with the mesenchymal lineage precursor or stem cells.

The term "T-cell" as used herein refers to a thymus-derived cell that participates in a variety of cell-mediated immune reactions.

The term "stimulation" as used herein means a primary response induced by binding of a stimulatory molecule (e.g., a TCR/CD3 complex) with its cognate ligand thereby mediating a signal transduction event, such as, but not limited to, signal transduction via the TCR/CD3 complex. Stimulation can mediate altered expression of certain molecules, such as downregulation of TGF-β, and/or reorganization of cytoskeletal structures, and the like.

The term "activation" as used herein refers to the state of a T cell that has been sufficiently stimulated to induce detectable cellular proliferation. Activation can also be associated with induced cytokine production, and detectable effector functions. The term "activated T cells" refers to, among other things, T cells that are undergoing cell division.

T cells that have recently been activated will typically express a series of activation markers at different time points following activation. Activation markers include receptors such as chemokine and cytokine receptors, adhesion molecules, co-stimulatory molecules, and MHC-class II proteins. Flow cytometry can be used to evaluate various types of surface or intracellular markers that indicate the activation status of T cells. Two of the most commonly used immediate early activation markers for assessing the activation status of human peripheral blood mononuclear (PBMC) T cells are CD69 and CD40L.

CD69 (AIM, Leu23, MLR3) is a signaling membrane glycoprotein involved in inducing T cell proliferation. CD69 is typically expressed at very low levels on resting CD4+ or CD8+ T cells in PBMC (<5-10%), and is one of the earliest assessable activation markers, being rapidly upregulated on CD4+ or CD8+ T cells within 1 hour of TCR stimulation or other T cell activators such as phorbol esters via a protein kinase C (PKC) dependent pathway. Expression of CD69 typically peaks by 16-24 hours and then declines, being barely detectable 72 hours after the stimulus has been withdrawn.

CD40L (CD154) is a member of the TNF-receptor superfamily that functions as a co-stimulatory molecule by binding CD40 which is constitutively expressed on antigen presenting cells (APCs). The CD40L-CD40 ligation results in the activation of multiple downstream pathways including the MAPK (JNK, p38, ERK1/2), NF-κB, and STAT3 transcription factors. CD40L expression is quickly upregulated within 1-2 hours after TCR stimulation via the transcription factors NFAT and AP-1. CD40L expression peaks near 6 hours after stimulation, and declines by 16-24hrs. CD40L expression however is biphasic, and the addition of anti-CD28 or IL-2 along with TCR stimulation typically leads to sustained expression for several days.

The term "specifically binds" as used herein means a ligand, for example, an antibody, which recognizes and binds with a cognate binding partner (e.g., a stimulatory and/or costimulatory molecule present on a T cell) present in a sample, but does not substantially recognize or bind other molecules in the sample.

The term "stimulatory ligand" as used herein, means a ligand that can specifically bind with a cognate binding partner (referred to herein as a "stimulatory molecule") on a T cell, thereby mediating a primary response by the T cell. Stimulatory ligands are well-known in the art and encompass, *inter alia,* an MHC Class I molecule loaded with a peptide, an anti-CD3 antibody, a superagonist anti-CD28 antibody, and a superagonist anti-CD2 antibody. The stimulatory ligand may be used in soluble form, expressed or attached to the surface of a cell or immobilized on a surface.

The term "superagonist antibody" as used herein, means an antibody that specifically binds with a molecule on a T cell and can mediate a primary activation signal event in a T cell without interaction of the TCR/CD3 complex or CD2 on the T cell. Exemplary superagonist antibodies include, but are not limited to, a superagonist anti-CD28 antibody and a superagonist anti-CD2 antibody. Unless referred to as a "superagonist", an anti-CD2 antibody, or an anti-CD28 antibody, and the like, is a co-stimulatory ligand as defined elsewhere herein, and provides a co-stimulatory signal rather than a primary activation signal.

The term "stimulatory molecule" as used herein, means a molecule on a T cell that specifically binds with a cognate stimulatory ligand.

The term "co-stimulatory signal" as used herein, refers to a signal, which in combination with a primary signal, such as TCR/CD3 ligation, mediates a T cell response, such as, but not limited to, activation, proliferation, differentiation into effector cells, induction of cytotoxicity or cytokine secretion.

The term "co-stimulatory ligand" as used herein, includes a molecule on an antigen presenting cell (APC) (e.g., a dendritic cell, B cell, and the like) or an artificial APC (aAPC) that specifically binds a cognate co-stimulatory molecule on a T cell, thereby providing a signal which, in addition to the primary signal provided by, for instance, binding of a TCR/CD3 complex with an MHC molecule loaded with peptide, mediates a T cell response, including, but not limited to, activation, proliferation, differentiation into effector cells, induction of cytotoxicity or cytokine secretion. A co-stimulatory ligand can include, but is not limited to, CD7, B7-1 (CD80), B7-2 (CD86), PD-L1, PD-L2, 4-1BBL, OX40L, inducible costimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, an agonist or antibody that binds Toll ligand receptor and a ligand that specifically binds with B7-H3. A co-stimulatory ligand also encompasses, *inter alia,* an antibody that specifically binds with a co-stimulatory molecule present on a T cell, such as, but not limited to, CD27, CD28, 4-1BB, OX40, CD30, CD40, PD-1, ICOS, lymphocyte function-associated antigen- 1 (LFA-1), CD2, CD7, LIGHT, NKG2C, B7-H3, and a ligand that specifically binds with CD83. These and other ligands are well-known in the art and have been well characterized as described in, for example, Schwartz et al., 2001; Schwartz et al., 2002; and Zhang et al., 2004. The skilled person would appreciate that a mutant or variant of a known ligand could be used and methods of producing such mutants and variants are well-known in the art.

The term "aAPC" as used herein includes, but is not limited to, cell-based aAPCs, bead-based APCs, microparticle aAPCs, and nanoparticle aAPCs. Materials which have been used include glass, poly (glycolic acid), poly(lactic-co-glycolic acid), iron-oxide, liposomes, lipid bilayers, sepharose, and polystyrene. The aAPC comprises a stimulatory ligand, for example, a stimulatory ligand that specifically binds with a TCR/CD3 complex such that a primary signal is transduced. The aAPC may further comprise at least one co-stimulatory ligand that specifically binds with at least one co-stimulatory molecule present on a T cell.

For the purposes for the present disclosure, the term "antibody" includes a protein capable of specifically binding to a stimulatory molecule on a T cell by virtue of an antigen binding domain contained within a Fv. This term includes four chain antibodies (e.g., two light (L) chains and two heavy (H) chains), recombinant or modified antibodies (e.g., chimeric antibodies, humanized antibodies, human antibodies, CDR-grafted antibodies, primatized antibodies, de-immunized antibodies, synhumanized antibodies, half-antibodies, bispecific antibodies).

As used herein, "variable region" refers to the portions of the light and/or heavy chains of an antibody as defined herein that is capable of specifically binding to an antigen and, includes amino acid sequences of complementarity determining regions (CDRs), that is, CDR1, CDR2, and CDR3, and framework regions (FRs). For example, the variable region comprises three or four FRs (e.g., FR1, FR2, FR3 and optionally FR4) together with three CDRs. V_{H} refers to the variable region of the heavy chain. V_{L} refers to the variable region of the light chain.

As used herein, the term "complementarity determining regions" (syn. CDRs, i.e., CDR1, CDR2, and CDR3) refers to the amino acid residues of an antibody variable region the presence of which are major contributors to specific antigen binding. Each variable region domain (V_{H} or V_{L}) typically has three CDR regions identified as CDR1, CDR2 and CDR3.

"Framework regions" (FRs) are those variable region residues other than the CDR residues.

As used herein, the term "Fv" shall be taken to mean any protein, whether comprised of multiple polypeptides or a single polypeptide, in which a V_{L} and a V_{H} associate and form a complex having an antigen binding domain that is capable of specifically binding to an antigen. The V_{H} and the V_{L} which form the antigen binding domain can be in a single polypeptide chain or in different polypeptide chains. Furthermore, a Fv of the disclosure (as well as any protein of the disclosure) may have multiple antigen binding domains which may or may not bind the same antigen. This term shall be understood to encompass fragments directly derived from an antibody as well as proteins corresponding to such a fragment produced using recombinant means. In some examples, the V_{H} is not linked to a heavy chain constant domain (C_{H}) 1 and/or the V_{L} is not linked to a light chain constant domain (C_{L}). Exemplary Fv containing polypeptides or proteins include a Fab fragment, a Fab' fragment, a F(ab') fragment, a scFv, a diabody, a triabody, a tetrabody or higher order complex, or any of the foregoing linked to a constant region or domain thereof, for example, C_{H}2 or C_{H}3 domain, for example, a minibody.

A "Fab fragment" consists of a monovalent antigen-binding fragment of an immunoglobulin, and can be produced by digestion of a whole antibody with the enzyme papain, to yield a fragment consisting of an intact light chain and a portion of a heavy chain or can be produced using recombinant means.

A "Fab' fragment" of an antibody can be obtained by treating a whole antibody with pepsin, followed by reduction, to yield a molecule consisting of an intact light chain and a portion of a heavy chain comprising a V_{H} and a single constant domain. Two Fab' fragments are obtained per antibody treated in this manner. A Fab' fragment can also be produced by recombinant means.

A "F(ab')₂ fragment" of an antibody consists of a dimer of two Fab' fragments held together by two disulfide bonds, and is obtained by treating a whole antibody molecule with the enzyme pepsin, without subsequent reduction.

A "Fab₂" fragment is a recombinant fragment comprising two Fab fragments linked using, for example, a leucine zipper or a C_{H}3 domain.

A "single chain Fv" or "scFv" is a recombinant molecule containing the variable region fragment (Fv) of an antibody in which the variable region of the light chain and the variable region of the heavy chain are covalently linked by a suitable, flexible polypeptide linker.

### T cell stimulation

In one embodiment of the disclosure, the T cells may be stimulated by a single agent. In another embodiment, T cells are stimulated with two agents, one that induces a primary signal and a second that is a co-stimulatory signal.

Ligands useful for stimulating a single signal or stimulating a primary signal and an accessory molecule that stimulates a second signal may be used in soluble form, expressed or attached to the surface of a cell or immobilized on a surface.

The surface may be any surface capable of having an agent/ligand bound thereto or integrated into and that is biocompatible, that is, substantially non-toxic to the target cells to be stimulated. The biocompatible surface may be biodegradable or non-biodegradable. The surface may be natural or synthetic, and a synthetic surface may be a polymer.

An agent may be attached or coupled to, or integrated into a surface by a variety of methods known and available in the art. The agent may be a natural ligand, a protein ligand, or a synthetic ligand. The attachment may be covalent or noncovalent, electrostatic, or hydrophobic and may be accomplished by a variety of attachment means, including for example, chemical, mechanical, enzymatic, electrostatic, or other means whereby a ligand is capable of stimulating the cells. For example, the antibody to a ligand first may be attached to a surface, or avidin or streptavidin may be attached to the surface for binding to a biotinylated ligand. The antibody to the ligand may be attached to the surface via an anti-idiotype antibody. Another example includes using protein A or protein G, or other non-specific antibody binding molecules, attached to surfaces to bind an antibody. Alternatively, the ligand may be attached to the surface by chemical means, such as cross-linking to the surface, using commercially available cross-linking reagents (Pierce, Rockford, IL) or other means.

The amount of a particular ligand attached to a surface may be readily determined by flow cytometric analysis if the surface is that of beads or determined by enzyme-linked immunosorbent assay (ELISA) if the surface is a tissue culture dish, mesh, fibers, bags, for example.

When coupled to a surface, the agents may be coupled to the same surface (i.e., in "cis" formation) or to separate surfaces (i.e., in "trans" formation). Alternatively, one agent may be coupled to a surface and the other agent in solution. In one embodiment, the agent providing the co-stimulatory signal is bound to a cell surface and the agent providing the primary activation signal is in solution or coupled to a surface. In a preferred embodiment, the two agents are immobilized on beads, either on the same bead, i.e., "cis," or to separate beads, i.e., "trans."

In one embodiment, the molecule providing the primary activation signal is a CD3 ligand, and the co-stimulatory molecule is a CD28 ligand. In a preferred embodiment, the CD3 ligand is an anti-CD3 antibody or a fragment thereof and the CD28 ligand is an anti-CD28 antibody or a fragment thereof. In one embodiment, the anti-CD3 antibody or fragment thereof and the anti-CD28 antibody of fragment thereof are used in soluble form. In an alternate embodiment, one or both of the anti-CD3 antibody or fragment thereof and the anti-CD28 antibody of fragment thereof are immobilized onto a surface. In one embodiment, both the anti-CD3 antibody or fragment thereof and the anti-CD28 antibody of fragment are co-immobilized on the same surface, for example, beads.

In one embodiment, the ratio of CD3:CD28 antibody bound to the beads ranges from 100:1 to 1:100 and all integer values there between. In one aspect of the present invention, more anti-CD28 antibody is bound to the particles than anti-CD3 antibody, i.e. the ratio of CD3:CD28 is less than one. In certain embodiments of the invention, the ratio of anti CD28 antibody to anti CD3 antibody bound to the beads is greater than 2:1. In one particular embodiment, a 1:100 CD3:CD28 ratio of antibody bound to beads is used. In another embodiment, a 1:75 CD3:CD28 ratio of antibody bound to beads is used. In a further embodiment, a 1:50 CD3:CD28 ratio of antibody bound to beads is used. In another embodiment, a 1:30 CD3:CD28 ratio of antibody bound to beads is used. In one preferred embodiment, a 1:10 CD3:CD28 ratio of antibody bound to beads is used. In another embodiment, a 1:3 CD3:CD28 ratio of antibody bound to the beads is used. In yet another embodiment, a 3:1 CD3:CD28 ratio of antibody bound to the beads is used.

The skilled person will appreciate that the agent(s) used for stimulation of the T cells is provided in an amount sufficient to mediate a T cell response, such as, but not limited to, activation, proliferation, differentiation into effector cells, induction of cytotoxicity or cytokine secretion. Preferably, the agent(s) used for stimulation of the T cells is provided in an amount sufficient to mediate T cell proliferation.

### Sources of T cells

Prior to stimulation/activation, a source of T cells is obtained from a subject. The term "subject" is intended to include living organisms in which an immune response can be elicited (e.g., mammals). Examples of subjects include humans, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available in the art, may be used. In certain embodiments of the present invention, T cells can be obtained from a unit of blood collected from a subject using any number of techniques known to the skilled person, such as ficoll separation. In one preferred embodiment, cells from the circulating blood of an individual are obtained by apheresis or leukapheresis. The apheresis product typically contains lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing steps. In one embodiment, the cells are washed with phosphate buffered saline (PBS). In an alternative embodiment, the wash solution lacks calcium and may lack magnesium or may lack many if not all divalent cations. As those of ordinary skill in the art would readily appreciate a washing step may be accomplished by methods known to those in the art, such as by using a semi- automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

Enrichment of a T cell population by negative selection can be accomplished with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for T cells (CD3+) by negative selection, a monoclonal antibody cocktail typically includes antibodies specific for B cells (CD19), monocytes (CD14), NK cells (CD56), etc. The antibodies are typically immobilized on a surface (e.g., particles such as beads).

For isolation of a desired population of cells by positive or negative selection, the concentration of cells to beads can be varied. In certain embodiments, it may be desirable to significantly decrease the volume in which beads and cells are mixed together (i.e., increase the concentration of cells), to ensure maximum contact of cells and beads. For example, in one embodiment, a concentration of 2 billion cells/ml is used. In one embodiment, a concentration of 1 billion cells/ml is used. In a further embodiment, greater than 100 million cells/ml is used. In a further embodiment, a concentration of cells of 10, 15, 20, 25, 30, 35, 40, 45, or 50 million cells/ml is used. In yet another embodiment, a concentration of cells from 75, 80, 85, 90, 95, or 100 million cells/ml is used. In further embodiments, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells.

If desired or necessary, monocyte populations (i.e., CD14⁺ cells) may be depleted from blood preparations prior to co-culture with the mesenchymal lineage precursor or stem cells or ex vivo expansion by a variety of methodologies, including anti-CD14 coated beads or columns, or utilization of the phagocytotic activity of these cells to facilitate removal, or by the use of counterflow centrifugal elutriation. Accordingly, in one embodiment, the invention uses paramagnetic particles of a size sufficient to be engulfed by phagocytotic monocytes. In certain embodiments, the paramagnetic particles are commercially available beads, for example, those produced by Dynal AS under the trade name Dynabeads^{™}. Exemplary Dynabeads^{™} in this regard are M-280, M-450, and M-500. In one aspect, other non- specific cells are removed by coating the paramagnetic particles with "irrelevant" proteins (e.g., serum proteins or antibodies). Irrelevant proteins and antibodies include those proteins and antibodies or fragments thereof that do not specifically target the T cells to be expanded. In certain embodiments the irrelevant beads include beads coated with sheep anti-mouse antibodies, goat anti-mouse antibodies, and human serum albumin.

### Expansion of T cells

The stimulated or activated T cells may be further expanded in cell culture using methods generally known in the art.

In one embodiment, the medium used to expand the T cells comprises an agent that can stimulate CD3 and an agent that can stimulate CD28 on the T cell.

### Co-culture of mesenchymal lineage precursor or stem cells with stimulated or activated T cells

The potency assay of the invention, measures the inhibition of T cell IL-2Rα expression following co-culture of T cells with the mesenchymal lineage precursor or stem cells. Inhibition of IL-2Rα expression is associated with a suppressive effect on T cell proliferation. Although not wanting to be limited by theory, the skilled person will appreciate that the mesenchymal lineage precursor or stem cells may inhibit or suppress T cell stimulation and/or activation and thereby, suppress T cell proliferation or they may act to suppress proliferation of activated T cells.

In an embodiment, the T cells are co-cultured with mesenchymal lineage precursor or stem cells in a culture medium comprising at least one T cell stimulating agent, preferably at a concentration capable of stimulating and/or activating the T cells. In another embodiment, the T cells are first stimulated and/or activated prior to co-culture with the mesenchymal lineage precursor or stem cells.

In an embodiment, the mesenchymal lineage precursor or stem cells are co-cultured with PBMC.

In an embodiment, mesenchymal lineage precursor or stem cells are co-cultured with PBMC in a culture medium comprising an agent that can stimulate CD3 and an agent that can stimulate CD28 on T cells, such as an antibody to CD3 and an antibody to CD28, for example, mouse anti-human CD3 and mouse anti-human CD28. In an embodiment, the antibody to CD3 and/or the antibody to CD28 is added to the culture medium in soluble form, each at a concentration of about 2µg/ml.

In an embodiment, the PBMC are co-cultured with mesenchymal precursor or stem cells at a ratio of 5 PBMC:1 mesenchymal precursor or stem cell. For example, 1 ×10⁶ PBMCs could be co-cultured with 2×10⁵ cells expanded from an enriched population of mesenchymal lineage precursor or stem cells. In a further embodiment, the cells a co-cultured in a final volume of 1ml.

In an embodiment, the isolated or enriched mesenchymal lineage precursor or stem cells are first expanded *ex vivo* or *in vitro* by culture and subsequently co-cultured with PBMC.

In an alternative embodiment, the isolated or enriched or cultured mesenchymal lineage precursor or stem cells are co-cultured with an enriched and/or expanded population of T cells.

It is preferred that the mesenchymal lineage precursor or stem cells are co-cultured with T-cells in culture medium comprising one or more T-cell stimulating agents/ligands. The skilled person would appreciate that the T-cells can be first stimulated and/or activated and then co-cultured with the mesenchymal lineage precursor or stem cells in the presence or absence of at least one T cell stimulating agent.

### Determining the amount of IL-2Rα levels

The present disclosure contemplates any form of assay, including Western blot, enzyme-linked immunosorbent assay (ELISA), fluorescence-linked immunosorbent assay (FLISA), competition assay, radioimmunoassay, lateral flow immunoassay, flow-through immunoassay, electrochemiluminescent assay, nephelometric-based assays, turbidometric-based assay, fluorescence activated cell sorting (FACS)-based assays for detection of TGFβ1 in culture medium used to culture mesenchymal lineage or precursor cells, and surface plasmon resonance (SPR or Biacore).

Following co-incubation of the mesenchymal lineage precursor or stems cells and T cells, the cells can be collected and lysed using well-known methods in the art. The cell lysates can then be assayed for the presence of IL-2Rα by, for example, ELISA or FLISA. Alternatively, the level of IL-2Rα expression may be determined by assaying intact cells by, for example, flow cytometry.

One form of a suitable assay is, for example, an ELISA or FLISA.

In one form, such an assay involves immobilizing a IL-2Rα binding protein onto a solid matrix, such as, for example a polystyrene or polycarbonate microwell or dipstick, a membrane, or a glass support (e.g., a glass slide). A test sample is then brought into direct contact with the IL-2Rα binding protein and IL-2Rα in the sample is bound or captured. Following washing to remove any unbound protein in the sample, a protein that binds to IL-2Rα at a distinct epitope is brought into direct contact with the captured IL-2Rα. This detector protein is generally labeled with a detectable reporter molecule, such as, for example, an enzyme (e.g. horseradish peroxidase (HRP)), alkaline phosphatase (AP) or β-gatactosidase) in the case of an ELISA or a fluorophore in the case of a FLISA. Alternatively, a second labeled protein can be used that binds to the detector protein. Following washing to remove any unbound protein the detectable reporter molecule is detected by the addition of a substrate in the case of an ELISA, such as, for example, hydrogen peroxide, TMB, or toluidine, or 5-bromo-4-chloro-3-indol-beta-D-galactopyranoside (x-gal). Of course, the immobilized (capture) protein and the detector protein may be used in the opposite manner.

The level of IL-2Rα in the sample is then determined using a standard curve that has been produced using known quantities of the marker or by comparison to a control sample.

In an embodiment, the inhibition of IL-2Rα expression is measured by comparing the level of IL-2Rα expression of a population of cells comprising T cells to the level of IL-2Rα of a population of cells following co-culture of a population of cells comprising T cells and a population of cells comprising mesenchymal lineage precursor or stem cells, and the difference expressed as "percentage inhibition".

The assays described above are readily modified to use chemiluminescence or electrochemiluminescence as the basis for detection.

As will be apparent to the skilled person, other detection methods based on an immunosorbent assay are useful in the performance of the present disclosure. For example, an immunosorbent method based on the description above using a radiolabel for detection, or a gold label (e.g., colloidal gold) for detection, or a liposome, for example, encapsulating NAD+ for detection or an acridinium linked immunosorbent assay.

In some examples of the disclosure, the level of IL-2Rα is determined using a surface plasmon resonance detector (e.g., BIAcore^{™}, GE Healthcare, Piscataway, N.J.), a flow through device (e.g., as described in US patent 7205159), a micro- or nano-immunoassay device (e.g., as described in US patent 7271007), a lateral flow device (e.g., as described in US publication 20040228761 or US publication 20040265926), a fluorescence polarization immunoassay (FPIA, e.g., as described in US patent 4593089 or US patent 4751190), or an immunoturbidimetric assay (e.g., as described in US patent 5571728 or US patent 6248597).

### Determining the amount of TNFR1 levels

The potency assay of the disclosure may also include the step of determining expression of TNFR1 by the mesenchymal lineage precursor or stem cells. The TNFR1 may be souble TNFR1 (sTNFR1). This step may be performed following co-culture of the mesenchymal lineage precursor or stem cells and T cells. Alternatively, TNFR1 expression may be measured in cell lysates of isolated or enriched or expanded mesenchymal lineage precursor or stem cells prior to co-culture with T cells. In an embodiment, TNFR1 expression is measured in cell lysates of cryopreserved enriched and/or expanded mesenchymal lineage precursor or stem cells.

The skilled person will appreciate that the methods described above for the detection of IL-2Rα expression can also be employed to detect TNFR1 expression. In a preferred embodiment, cell lysates as assayed by ELISA or FLISA.

In one form, such an assay involves immobilizing a TNFR1 binding protein onto a solid matrix. A test sample is then brought into direct contact with the TNFR1 binding protein and TNFR1 in the sample is bound or captured. Following washing to remove any unbound protein in the sample, a protein that binds to TNFR1 at a distinct epitope is brought into direct contact with the captured TNFR1. This detector protein is generally labeled as described above. Alternatively, a second labeled protein can be used that binds to the detector protein. Following washing to remove any unbound protein the detectable reporter molecule is detected by the addition of a substrate in the case of an ELISA as described above. The level of TNFR1 in the sample is then determined using a standard curve that has been produced using known quantities of the marker or by comparison to a control sample.

In an embodiment, TNFR1 expression is measured in cell lysates of cryopreserved enriched and/or expanded mesenchymal lineage precursor or stem cells, and at least 100 pg/mL TNFR1 is indicative of biological activity or therapeutic efficacy.

### Compositions and administration

A composition comprising mesenchymal lineage precursor or stem cells may be prepared in a pharmaceutically acceptable carrier. The term "pharmaceutically acceptable carrier" as used herein refers to compositions of matter that facilitate the storage, administration, and/or maintain the biological activity of the mesenchymal lineage precursor or stem cells.

In one example, the carrier does not produce significant local or systemic adverse effect in the recipient. The pharmaceutically acceptable carrier may be solid or liquid. Useful examples of pharmaceutically acceptable carriers include, but are not limited to, diluents, solvents, surfactants, excipients, suspending agents, buffering agents, lubricating agents, adjuvants, vehicles, emulsifiers, absorbants, dispersion media, coatings, stabilizers, protective colloids, adhesives, thickeners, thixotropic agents, penetration agents, sequestering agents, scaffolds, isotonic and absorption delaying agents that do not affect the viability and activity of the mesenchymal lineage precursor or stem cells. The selection of a suitable carrier is within the skill of those skilled in the art.

Compositions of the disclosure may conveniently be presented in unit dosage form and may be prepared by any of the methods well known in the art. The term "dosage unit form" as used herein refers to physically discrete units suited as unitary dosages for subjects to be treated; each unit containing a predetermined quantity of active compound calculated to produce the desired therapeutic or prophylactic effect in association with the pharmaceutical carrier. The dose of mesenchymal lineage precursor or stem cells may vary according to factors such as the disease state, age, sex, and weight of the subject to be treated.

The term "subject" refers to an animal, preferably a mammal including a non-primate (e.g., a cow, pig, horse, cat, dog, rat, or mouse) and a primate (e.g., a monkey, or a human). In a preferred embodiment, the subject is a human.

Exemplary doses include at least about 1 × 10⁶ cells. For example, a dose can comprise between about 1.0 × 10⁶ to about 1×10¹⁰ cells, for example, between about 1.1 × 10⁶ to about 1×10⁹ cells, for example, between about 1.2 × 10⁶ to about 1 × 10⁸ cells, for example, between about 1.3 × 10⁶ to about 1 × 10⁷ cells, for example, between about 1.4 × 10⁶ to about 9 × 10⁶ cells, for example, between about 1.5 × 10⁶ to about 8 × 10⁶ cells, for example, between about 1.6 × 10⁶ to about 7 × 10⁶ cells, for example, between about 1.7 × 10⁶ to about 6 × 10⁶ cells, for example, between about 1.8 × 10⁶ to about 5 × 10⁶ cells, for example, between about 1.9 × 10⁶ to about 4 × 10⁶ cells, for example, between about 2 × 10⁶ to about 3 × 10⁶ cells.

In one example, the dose comprises between about 5 × 10⁵ to 2 ×10⁷ cells, for example, between about 6 × 10⁶ cells to about 1.8 × 10⁷ cells. The dose may be, for example, about 6 × 10⁶ cells or about 1.8 × 10⁷ cells.

The mesenchymal lineage precursor or stem cells comprise at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or at least about 95% of the cell population of the composition.

Compositions of the disclosure may be cryopreserved. Cryopreservation of mesenchymal lineage precursor or stem cells can be carried out using slow-rate cooling methods or 'fast' freezing protocols known in the art. Preferably, the method of cryopreservation maintains similar phenotypes, cell surface markers and growth rates of cryopreserved cells in comparison with unfrozen cells.

The cryopreserved composition may comprise a cryopreservation solution. The pH of the cryopreservation solution is typically 6.5 to 8, preferably 7.4.

The cyropreservation solution may comprise a sterile, non-pyrogenic isotonic solution such as, for example, PlasmaLyte A^{™}. 100 mL of PlasmaLyte A^{™} contains 526 mg of sodium chloride, USP (NaCI); 502 mg of sodium gluconate (C₆H₁₁NaO₇); 368 mg of sodium acetate trihydrate, USP (C₂H₃NaO₂•3H₂O); 37 mg of potassium chloride, USP (KCI); and 30 mg of magnesium chloride, USP (MgCl₂•6H₂O). It contains no antimicrobial agents. The pH is adjusted with sodium hydroxide. The pH is 7.4 (6.5 to 8.0).

The cryopreservation solution may comprise Profreeze^{™}. The cryopreservation solution may additionally or alternatively comprise culture medium.

To facilitate freezing, a cryoprotectant such as, for example, dimethylsulfoxide (DMSO), is usually added to the cryopreservation solution. Ideally, the cryoprotectant should be nontoxic for cells and patients, nonantigenic, chemically inert, provide high survival rate after thawing and allow transplantation without washing. However, the most commonly used cryoprotector, DMSO, shows some cytotoxicity . Hydroxylethyl starch (HES) may be used as a substitute or in combination with DMSO to reduce cytotoxicity of the cryopreservation solution.

The cryopreservation solution may comprise one or more of DMSO, hydroxyethyl starch, human serum components and other protein bulking agents. In one example, the cryopreserved solution comprises about 5% human serum albumin (HSA) and about 10% DMSO. The cryopreservation solution may further comprise one or more of methycellulose, polyvinyl pyrrolidone (PVP) and trehalose.

In one embodiment, cells are suspended in 42.5% Profreeze^{™}/50% αMEM/7.5% DMSO and cooled in a controlled-rate freezer.

The cryopreserved composition may be thawed and administered directly to the subject or added to another solution, for example, comprising HA. Alternatively, the cryopreserved composition may be thawed and the mesenchymal lineage precursor or stem cells resuspended in an alternate carrier prior to administration.

Compositions of the disclosure can be administered by a route that is suitable for the particular disease state to be treated. For example, compositions of the disclosure can be administered systemically, i.e., parenterally, intravenously or by injection. Compositions of the disclosure can be targeted to a particular tissue or organ.

Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage.

In some embodiments, it may not be necessary or desirable to immunosuppress a patient prior to initiation of therapy with cellular compositions. Indeed, transplantation of allogeneic STRO-1+ cells in sheep was well tolerated in the absence of immunosuppression. However, in other instances it may be desirable or appropriate to pharmacologically immunosuppress a patient prior to initiating cell therapy. This may be accomplished through the use of systemic or local immunosuppressive agents, or it may be accomplished by delivering the cells in an encapsulated device. The cells may be encapsulated in a capsule that is permeable to nutrients and oxygen required by the cell and therapeutic factors the cell is yet impermeable to immune humoral factors and cells. Preferably the encapsulant is hypoallergenic, is easily and stably situated in a target tissue, and provides added protection to the implanted structure. These and other means for reducing or eliminating an immune response to the transplanted cells are known in the art. As an alternative, the cells may be genetically modified to reduce their immunogenicity.

It will be appreciated that the mesenchymal lineage precursor or stem cells may be administered with other beneficial drugs or biological molecules (growth factors, trophic factors). When administered with other agents, they may be administered together in a single pharmaceutical composition, or in separate pharmaceutical compositions, simultaneously or sequentially with the other agents (either before or after administration of the other agents). Bioactive factors which may be co-administered include anti-apoptotic agents (e.g., EPO, EPO mimetibody, TPO, IGF-I and IGF-II, HGF, caspase inhibitors); anti-inflammatory agents (e.g., p38 MAPK inhibitors, TGF-beta inhibitors, statins, IL-6 and IL-1 inhibitors, PEMIROLAST^{™}, TRANILAST^{™}, REMICADE^{™}, SIROLIMUS^{™}, and non-steroidal anti-inflammatory drugs (NSAIDs) such as TEPOXALIN^{™}, TOLMETIN^{™}, SUPROFEN^{™}); immunosupressive/immunomodulatory agents (e.g., calcineurin inhibitors such as cyclosporine, tacrolimus); mTOR inhibitors (e.g., SIROLIMUS^{™}, EVEROLIMUS^{™}); anti-proliferatives (e.g., azathioprine, mycophenolate mofetil); corticosteroids (e.g., prednisolone, hydrocortisone); antibodies such as monoclonal anti-IL-2Ralpha receptor antibodies (e.g., basiliximab, daclizumab), polyclonal anti-T-cell antibodies (e.g., anti-thymocyte globulin (ATG); antilymphocyte globulin (ALG); monoclonal anti-T cell antibody OKT3)); anti-thrombogenic agents (e.g., heparin, heparin derivatives, urokinase, PPack (dextrophenylalanine proline arginine chloromethylketone), antithrombin compounds, platelet receptor antagonists, anti-thrombin antibodies, anti-platelet receptor antibodies, aspirin, dipyridamole, protamine, hirudin, prostaglandin inhibitors, and platelet inhibitors); and anti-oxidants (e.g., probucol, vitamin A, ascorbic acid, tocopherol, coenzyme Q-10, glutathione, L-cysteine, N-acetylcysteine) as well as local anesthetics.

### Graft versus Host Disease and its Staging

Acute and chronic graft versus host disease (GVHD) are multisystem disorders that are complications of allogenic hematopoietic cell transplantation (usually in the form of a bone marrow or peripheral blood stem cell harvest). GVHD occurs when immune cells transplanted from a non-identical donor (the graft) recognize the transplant recipient (the host) as foreign, thereby initiating an immune reaction that causes disease in the transplant recipient. Clinical manifestations of acute GVHD include a classic maculopapular rash, persistent nausea and/or emesis; abdominal cramps with diarrhea; and a rising serum bilirubin concentration. In contrast, patients with chronic GVHD commonly demonstrate skin involvement resembling lichen planus or the cutaneous manifestations of scleroderma; dry oral mucosa with ulcerations and sclerosis of the gastrointestinal tract; and a rising serum bilirubin concentration.

GVHD has been classically divided into acute and chronic variants based upon the time of onset using a cut-off of 100 days. However, this conventional division has been challenged by the recognition that signs of acute and chronic GVHD may occur outside of these designated periods. This observation has led to the increased use of clinical findings, rather than a set time period, to differentiate between acute and chronic GVHD. The widely accepted National Institutes of Health (NIH) consensus criteria used to diagnose GVHD classify manifestations of GVHD as "diagnostic" or "distinctive" of chronic GVHD or as common to both acute and chronic GVHD (Filipovich AH et al. (2005) Biol Blood Marrow Transplant 11:945).

Patients with GVHD are sub-classified based upon the timing of presentation and the features present:
- Classic acute GVHD - Cases present within 100 days of hematopoietic cell transplant (HCT) and display features of acute GVHD. Diagnostic and distinctive features of chronic GVHD are absent.
- Persistent, recurrent, late onset acute GVHD - Cases present greater than 100 days post-HCT with features of acute GVHD. Diagnostic and distinctive features of chronic GVHD are absent.
- Classic chronic GVHD - Cases may present at any time post-HCT. Diagnostic and distinctive features of chronic GVHD are present. There are no features of acute GVHD.
- Overlap syndrome - Cases may present at any time post-HCT with features of both chronic GVHD and acute GVHD. On occasion, this is colloquially referred to as "acute on chronic" GVHD.

The pathophysiology of acute GVHD has been well described and extensively reviewed (Ferrara JL et al. (2006) Semin Hematol 43(1):3-10). Briefly, the events leading to the development of clinically apparent GVHD begin during pretransplant conditions, during which transplant recipients receive high-dose chemotherapy and/or radiotherapy. The tissue damage that occurs as a result of high-dose therapy results in activation of host antigen-presenting cells (APCs), upregulation of major histocompatibility antigen on the APC surface and presentation of host antigens. Donor T lymphocytes, infused with the stem cell graft, respond to antigenic differences in this milieu by clonal expansion, tissue migration and direct cell-cell cytotoxicity. High levels of pro-inflammatory cytokines, particularly tumour necrosis factor alpha (TNF-α), interleukin-1 (IL-1) and interleukin-2 (IL-2) and abundant host antigen lead to an inflammatory cascade that may result in severe tissue damage, organs dysfunction and death.

Clinically significant acute GVHD occurs in patients who receive an allogeneic hematopoietic cell transplant (HCT) despite intensive prophylaxis with immunosuppressive agents. The exact incidence of acute GVHD after allogeneic HCT is unknown. Reported incidence rates range from 9 to 50 percent in patients who receive an allogeneic HCT from a genotypically HLA-identical sibling (Lee SE et al. (2013) Bone Marrow Transplant 48:587).

Acute GVHD is also common in matched unrelated donors and in haploidentical related donors.

Numerous studies have identified the following risk factors for the development of acute graft-versus-host disease (GVHD) (Hahn T et al. (2008) J Clin Oncol 26:5728):
- Degree of HLA disparity (HLA mismatch or unrelated donor);
- Donor and recipient gender disparity (female donor to male recipient);
- Intensity of the transplant conditioning regimen;
- Acute GVHD prophylactic regimen used; and
- Source of graft (peripheral blood or bone marrow greater than umbilical cord blood).

Less well established risk factors include increasing age of the host, the cytomegalovirus (CMV) status of the donor and host, donor Epstein-Barr virus (EBV) seropositivity (Styczynski J et al. (2016) J Clin Oncol 34:2212); peripheral blood stem cell versus bone marrow transplantation, the presence of a sterile environment (including gut decontamination), and particular HLA haplotype. However, risk factors for acute GVHD differ by underlying disease, requiring distinct risk models for each condition (Hahn T et al. (2008) J Clin Oncol 26:5728).

GVHD is a common complication of allogeneic hematopoietic cell transplant (HCT) that classically presents in the early post-transplantation period. The initial signs and symptoms of acute GVHD most commonly occur around the time of white blood cell engraftment. Although initial definitions of acute GVHD required an onset of symptoms before 100 days post transplantation, the current National Institutes of Health (NIH) consensus criteria use clinical findings, rather than a set time period, to differentiate between acute and chronic GVHD. As such, patients presenting with typical findings of acute GVHD prior to day 100 are considered to have "classic acute GVHD," whereas patients presenting with the same findings after day 100, typically upon reduction of immunosuppression, are categorized as having "late onset acute GVHD" (Vigorito AC et al. (2009) Blood 114:702). Some clinicians also use the terms "early onset acute GVHD" or "hyperacute GVHD" to describe symptoms of acute GVHD occurring within 14 days of transplant (Sullivan KM et al. (1986) Blood 67:1172).

### Organ involvement

The skin, gastrointestinal tract, and liver are the principal target organs in patients with acute GVHD. In most patients, the first (and most common) clinical manifestation of acute GVHD is a maculopapular rash, usually occurring at or near the time of the white blood cell engraftment. The rash initially involves the nape of the neck, ears, shoulders, the palms of the hands, and the soles of the feet. It can be described as a sunburn and may be pruritic or painful. Histologic examination of the skin reveals changes in the dermal and epidermal layers (Sale GE et al. (1977) Am J Pathol 89:621). Characteristic findings include exocytosed lymphocytes, dyskeratotic epidermal keratinocytes, follicular involvement, satellite lymphocytes adjacent to or surrounding dyskeratotic epidermal keratinocytes, and dermal perivascular lymphocytic infiltration (Darmstadt GL et al. (1992) J Invest Dermatol 99:397). The stage of skin involvement is combined with information regarding the stage of gastrointestinal tract and liver involvement to determine the overall severity grade of acute GVHD.

Acute GVHD frequently involves both the upper and lower gastrointestinal tract. Gastrointestinal involvement usually presents with diarrhea and abdominal pain, but may also manifest as nausea, vomiting, and anorexia. Confirmation of the diagnosis is provided by pathologic evaluation of tissue obtained by upper endoscopy, rectal biopsy or colonoscopy. The diagnosis of gastrointestinal involvement requires pathologic evaluation of the tissue. Once diagnosed, the degree of gastrointestinal involvement is graded based upon the severity of diarrhea: Stage 1 - Diarrhea 500 to 1000 mL/day; Stage 2 - Diarrhea 1000 to 1500 mL/day; Stage 3 - Diarrhea 1500 to 2000 mL/day; and Stage 4 - Diarrhea >2000 mL/day or pain or ileus.

Involvement of the lower gastrointestinal tract with acute GVHD is often severe, and is characterized by diarrhea, with or without hematochezia, and abdominal cramping. Confirmation of the diagnosis is performed by pathologic evaluation of tissue obtained by rectal biopsy or colonoscopy. Patients with acute GVHD can develop severe diarrhea, occasionally exceeding 10 litres a day. The stool may initially be watery, but frequently becomes bloody. The diarrhea is secretory and characteristically continues despite fasting and occurs day and night. It can be accompanied by crampy abdominal pain that can also be difficult to manage. Severe ileus may develop in association with acute GVHD or result from increased opioid use required to control the physical discomfort. A rectal biopsy is usually helpful in making the diagnosis of acute GVHD affecting the gastrointestinal tract. On histologic examination, crypt cell necrosis is observed with the accumulation of degenerative material in the dead crypts.

Involvement of the upper gastrointestinal tract with acute GVHD often presents with anorexia, dyspepsia, food intolerance, nausea, and vomiting (Weisdorf DJ et al. (1990) Blood 76:624). Patients may also display gingivitis and mucositis, although these findings are more commonly due to the effects of conditioning regimens. The diagnosis is verified by positive upper endoscopic biopsies of the esophagus and stomach. The differential diagnosis includes herpes simplex virus or candida esophagitis, gastritis, peptic ulcers, and gastrointestinal toxicity due to chemotherapy and/or radiation.

Liver involvement usually presents in patients with signs of cutaneous and/or gastrointestinal acute GVHD (Ratanatharathorn V et al. (1998) Blood 92:2303). Rarely, patients have moderate to severe hepatic GVHD without evidence of other organ involvement. Although liver involvement may be suggested by abnormalities in liver function tests in the setting of cutaneous or gastrointestinal GVHD, liver biopsy is required to document GVHD of the liver. Hepatic involvement is manifested by abnormal liver function tests, with the earliest and most common finding being a rise in the serum levels of conjugated bilirubin and alkaline phosphatase. Serum cholesterol is usually elevated, while coagulopathy and hyperammonemia are very rare but may develop in severe cases. Patients may also demonstrate painful hepatomegaly, dark urine, pale stool, fluid retention, and pruritus. Fever, anorexia, and nausea are common nonspecific symptoms. Although the concurrent presence of the characteristic rash provides suggestive clinical evidence, biopsy is the most definitive method to diagnose GVHD of the liver. However, this may not be feasible because of the possibility of acute bleeding due to severe thrombocytopenia soon after HCT.

### Biomarkers for diagnosis of acute GVHD

Use of serum biomarkers for the diagnosis of acute GVHD is an active area of investigation. Biomarkers or panels of biomarkers are generally used in combination with each other or with other findings. An ideal biomarker would be able to not only predict the appearance of clinical acute GVHD but also guide management. There are many candidate biomarkers but none are ready for clinical application.

One candidate biomarker is suppression of tumorigenicity 2 (ST2), which is a member of the interleukin-1 receptor family.

Analysis of the pattern of plasma and urine polypeptides using proteomics has shown promise in enabling early diagnosis of acute GVHD (Srinivasan R et a. (2006) Exp Hematol 34:796). As an example, it has been proposed that a panel of markers including Interleukin-2 receptor-alpha, tumour necrosis factor receptor-1, Interleukin-8, and hepatocyte growth factor can confirm the diagnosis of acute GVHD at the onset of clinical symptoms and provide prognostic information independent of GVHD severity (Paczesny S et al. (2009) Blood 113:273). Use of Reg3 has also been found useful for the diagnosis of acute gut GVHD (Ferrara JL et al. (2011) Blood 118:6702). Additionally, plasma levels of CD30 have been found to be elevated in patients with acute GVHD (Chen YB et al. (2012) Blood 120:691).

Analysis of the plasma microRNA signature might provide a noninvasive biomarker for acute GVHD. In one study, evaluation of a panel of six microRNAs was able to distinguish HCT recipients who had acute GVHD from patients without acute GVHD, and was able to predict the severity of acute GVHD (Xiao B et al. (2013) Blood 122:3365). Four were combined into a panel that was predictive of acute GVHD, and the levels of miRNA biomarkers were positively associated with acute GVHD severity. More importantly, those elevated miRNAs can be detected before onset of acute GVHD. These data are being validated in a larger cohort of patients.

### Staging of GVHD

Several systems for grading acute GVHD have been developed. The two most popular are the Glucksberg grade (I-IV) (Glucksberg H et al (1974) Transplantation 18(4):295) and the International Bone Marrow Transplant Registry (IBMTR) grading system (A-D) (Rowlings PA et al., (1997) Br J Hematol 97(4):855). The severity of acute GVHD is determined by an assessment of the degree of involvement of the skin, liver, and gastrointestinal tract. The stages of individual organ involvement are combined with (Glucksberg) or without (IBMTR) the patient's performance status to produce an overall grade, which has prognostic significance. Grade I(A) GVHD is characterized as mild disease, grade II(B) GVHD as moderate, grade III(C) as severe, and grade IV(D) life-threatening (Przepiorka D, Weisdorf D, Martin P, Klingemann HG, Beatty P, Hows J, Thomas ED (1995) Bone Marrow Transplant. 1995;15(6):825; Cahn JY et al. (2005) Blood 106(4):1495).

The IBMTR grading system defines the severity of acute GVHD as follows:
- Grade A - Stage 1 skin involvement alone (maculopapular rash over <25 percent of the body) with no liver or gastrointestinal involvement
- Grade B - Stage 2 skin involvement; Stage 1 to 2 gut or liver involvement
- Grade C - Stage 3 involvement of any organ system (generalized erythroderma; bilirubin 6.1 to 15.0 mg/dL; diarrhea 1500 to 2000 mL/day)
- Grade D - Stage 4 involvement of any organ system (generalized erythroderma with bullous formation; bilirubin >15 mg/dL; diarrhea >2000 mL/day OR pain OR ileus).

Grading is important in terms of assessing the response to prophylaxis or treatment, impact upon survival, and association with graft-versus-leukemia effect. Patients with moderate to severe GVHD have a significantly higher mortality rate compared with those with mild disease. As an example, estimated five-year survival rates of patients with grade III (C) and grade IV (D) acute GVHD are 25 and 5 percent, respectively (Przepiorka D et al. (1995) Bone Marrow Transplant 15:825). However, caution must be used when applying these estimated survival rates to current patient population given changes in post-HCT care. Current preventive regimens may alter overall outcomes and expressions of the disease. Typically initial grading of each organ is calculated within a 10-day window (-5 to +5 days) of initiation of steroid therapy. Real-time staging and grading is then determined weekly by the attending physician, supported by laboratory and clinical information and histologic confirmation when possible.

Recent studies (see for example, MacMillan ML et al (2010) Blood 115:5412-5417) have proposed that day 28 response, including PR and Cr, be incorporated as the early target, which can predict the later, and more critical outcomes for patients with acute GVHD.

### Hematopoietic Stem Cell Transplantation (HSCT)

An "hematopoietic stem cell transplantation (HSCT)" is a graft comprising multipotent hematopoietic stem cells which can be derived, for example, from bone marrow or peripheral blood. The transplant may include some non-stem cells, for example, APCs including DCs and/or lymphocytes.

"Hematopoietic stem cells" can self-renew and differentiate to give rise to all the blood cell types including myeloid (monocytes and macrophages, neutrophils, basophils, eosinophils, dendritic cells), erythroid (erythrocytes), megakaryocytic (platelets) and lymphoid lineages (T-cells, B-cells, NK-cells). Throughout differentiation, the hematopoietic stem cell first loses its self-renewal capacity, then loses lineage potential step by step as it commits to becoming a mature effector cell. Typically a Lin-, CD34+, CD38-, CD90+, CD45RA- human cell is a hematopoietic stem cell. In one example, expression of CD34 is used to identify hematopoietic stem cells in peripheral blood isolated from human donors.

HSCT can be used in the treatment of diseases and conditions which require stem cell transplants. For example, the stem cells can be used for the treatment of failure or dysfunction of normal blood cell production and maturation, hematopoietic malignancy, autoimmune disease, liver disease, or immunodeficiency (by reason of for example, irradiation, chemotherapy or infection with a pathogen).

The stem cells may be expanded or differentiated *ex vivo* prior to administration to a subject.

Allogeneic hematopoietic stem-cell transplantation may be used to treat one or more of the following conditions: acute myeloid leukemia, acute lymphoblastic leukemia, chronic myeloid leukemia, chronic lymphocytic leukemia, myeloproliferative disorders, myelodysplastic syndromes, multiple myeloma, non-Hodgkin lymphoma, Hodgkin disease, aplastic anemia, pure red cell aplasia, paroxysmal nocturnal hemoglobinuria, Fanconi anemia, Thalassemia major, sickle cell anemia, Severe combined immunodeficiency (SCID), Wiskott-Aldrich syndrome, hemophagocytic lymphohistiocytosis (HLH), inborn errors of metabolism (e.g., mucopolysaccharidosis,, Gaucher disease, metachromatic leukodystrophies and adrenoleukodystrophies).

### Prevention or Treatment of GVHD

The mesenchymal lineage precursor or stem cells and/or progeny cells thereof may be used in a method for prevention or treatment of a subject with GVDH that includes the step of administering the cells, in an effective dose for prevention or treatment, to the subject. In one embodiment, the subject has received one or more allogeneic hematopoietic stem cell grafts resulting in GVHD.

Symptoms of GVHD, include sclerotic skin, limitation of oral intake, dryness of eyes, gastrointestinal (GI) tract symtoms such as dysphagia, anorexia, nausea, vomiting, abdominal pain, or diarrhea, liver symptoms as manifested by elevated bilirubin, elevated alkaline phosphatase, and elevated alanine aminotranferease (ALT) /aspartate aminotransferase (AST) (AST/ALT) ratio, shortness of breath, and/or tightness of arms or legs.

A subject may exhibit multiple symptoms depending on the tissue that is affected by the graft-versus-host disease. Some subjects have 4-5 symptoms others may have 1-2 symptoms. The mesenchymal lineage precursor or stem cells and/or progeny cells thereof thereof may be used to treat one, or more, or all and all of the symptoms associated with GVHD in the subject.

The effective dose per injection of mesenchymal lineage precursor or stem cells and/or progeny cells thereof thereof for treatment of GVHD or the symptoms of GVHD in a mammal and in particular, a human may be between 1×10⁴ cells/kg body weight and 1×10⁸ cells/kg body weight; between 1×10⁴ cells/kg body weight and 1×10⁸ cells/kg body weight; between 2×10⁴ cells/kg body weight and 1×10⁸ cells/kg body weight; between 2.5×10⁴ cells/kg body weight and 1×10⁸ cells/kg body weight; between 2×10⁴ cells/kg body weight and 1×10⁷ cells/kg body weight; between 2.5×10⁴ cells/kg body weight and 1×10⁷ cells/kg body weight; between 2×10⁴ cells/kg body weight and 3×10⁶ cells/kg body weight; between 2.5×10⁴ cells/kg body weight and 3×10⁶ cells/kg body weight; between 2×10⁴ cells/kg body weight and 2×10⁶ cells/kg body weight; between 2.5×10⁴ cells/kg body weight and 2×10⁶ cells/kg body weight; between 2×10⁴ cells/kg body weight and 1×10⁶ cells/kg body weight; between 2.5×10⁴ cells/kg body weight and 1×10⁶ cells/kg body weight; between 2×10⁴ cells/kg body weight and 1×10⁵ cells/kg body weight; or between 2.5×10⁴ cells/kg body weight and 1×10⁵ cells/kg body weight.

The mesenchymal lineage precursor or stem cells and/or progeny cells thereof may be surgically implanted, injected, delivered (e.g., by way of a catheter or syringe), or otherwise administered directly or indirectly to the site in need of repair or augmentation, e.g., an organ or into the blood system of a subject.

In one embodiment, the mesenchymal lineage precursor or stem cells and/or progeny cells thereof are delivered to the blood stream of a subject. For example, the mesenchymal lineage precursor or stem cells and/or progeny cells thereof are delivered parenterally. Exemplary routes of parenteral administration include, but are not limited to, intravenous, intramuscular, subcutaneous, intra-arterial, intraperitoneal, intraventricular, intracerebroventricular, intrathecal.

In one embodiment, the mesenchymal lineage precursor or stem cells and/or progeny cells thereof are injected into the site of delivery, e.g., using a syringe or through a catheter or a central line.

Selecting an administration regimen for a therapeutic formulation depends on several factors, including the serum or tissue turnover rate of the entity, the level of symptoms, and the immunogenicity of the entity. Preferably, an administration regimen maximizes the amount of therapeutic compound delivered to the patient consistent with an acceptable level of side effects.

In one example, the mesenchymal lineage precursor or stem cells and/or progeny cells thereof are delivered as a single bolus dose. Alternatively, the mesenchymal lineage precursor or stem cells and/or progeny cells thereof are administered by continuous infusion.

Without being limited to any particular administration method, non-oral administration method is preferred. While whole body or partial body administration is possible, whole body administration is preferred and intravenous injection is most preferred.

The mesenchymal lineage precursor or stem cells and/or progeny cells thereof or composition comprising same can be used in combination with other active agent(s). For example, the mesenchymal lineage precursor or stem cells of the disclosure may be combined with corticosteroids, non-steroidal anti-inflammatory compounds, or other agents useful in treating inflammation. The combined use of the mesenchymal lineage precursor or stem cells with these other agents may be concurrent, or given sequentially, that is, the mesenchymal lineage precursor or stem cells or composition comprising same may be given prior to or after treatment with one or more other active agents.

In one embodiment, mesenchymal lineage precursor or stem cells and/or progeny cells thereof are administered prior to, simultaneously with, or after administration of hematopoietic stem cells.

In another embodiment, the hematopoietic stem cells are co-cultured with the mesenchymal lineage precursor or stem cells and/or progeny cells thereof prior to administration to the subject.

The attending physician may decide on the appropriate sequence of administering the mesenchymal lineage precursor or stem cells, or a composition comprising same, in combination with other active agents.

### Treatment of inflammatory disorders

The present disclosure also provides a method of treating an autoimmune disease in a subject. The method comprises administering to the subject, mesenchymal lineage precursor or stem cells or progeny thereof as described herein in an amount effective to treat the autoimmune disease in the subject. Autoimmune diseases which may be treated in accordance with the present disclosure include, but are not limited to, multiple sclerosis, Type 1 diabetes, rheumatoid arthritis, uveitis, celiac disease, lupus, autoimmune thyroid diseases, inflammatory bowel disease, autoimmune lymphoproliferative disease (ALPS) demyelinating disease, autoimmune encephalomyelitis, autoimmune gastritis (AIG), and autoimmune glomerular diseases.

### Genetically-modified cells

In one embodiment, the mesenchymal lineage precursor or stem cells are genetically modified, for example, to express and/or secrete a protein of interest, for example, a protein providing a therapeutic and/or prophylactic benefit.

Methods for genetically modifying a cell will be apparent to the skilled person. For example, a nucleic acid that is to be expressed in a cell is operably-linked to a promoter for inducing expression in the cell. For example, the nucleic acid is linked to a promoter operable in a variety of cells of a subject, such as, for example, a viral promoter, for example, a CMV promoter (e.g., a CMV-IE promoter) or a SV-40 promoter. Additional suitable promoters are known in the art.

Preferably, the nucleic acid is provided in the form of an expression construct. The term "expression construct" as used herein refers to a nucleic acid that has the ability to confer expression on a nucleic acid (e.g., a reporter gene and/or a counter-selectable reporter gene) to which it is operably connected, in a cell. Within the context of the present disclosure, it is to be understood that an expression construct may comprise or be a plasmid, bacteriophage, phagemid, cosmid, virus sub-genomic or genomic fragment, or other nucleic acid capable of maintaining and/or replicating heterologous DNA in an expressible format.

Methods for the construction of a suitable expression construct for performance of the invention will be apparent to the skilled person and are described, for example, in Ausubel F. M., 1987 including all updates untill present; or Sambrook & Green, 2012. For example, each of the components of the expression construct is amplified from a suitable template nucleic acid using, for example, PCR and subsequently cloned into a suitable expression construct, such as, for example, a plasmid or a phagemid.

Vectors suitable for such an expression construct are known in the art and/or described herein. For example, an expression vector suitable for the method of the present invention in a mammalian cell is, for example, a vector of the pcDNA vector suite (Invitrogen), a vector of the pCI vector suite (Promega), a vector of the pCMV vector suite (Clontech), a pM vector (Clontech), a pSI vector (Promega), a VP 16 vector (Clontech), or a vector of the pcDNA vector suite (Invitrogen).

The skilled person will be aware of additional vectors and sources of such vectors, such as, for example, Invitrogen Corporation, Clontech or Promega.

Means for introducing the isolated nucleic acid molecule or a gene construct comprising same into a cell for expression are known to those skilled in the art. The technique used for a given organism depends on the known successful techniques. Means for introducing recombinant DNA into cells include microinjection, transfection mediated by DEAE-dextran, transfection mediated by liposomes such as by using lipofectamine (Gibco, MD, USA) and/or cellfectin (Gibco, MD, USA), PEG-mediated DNA uptake, electroporation and microparticle bombardment such as by using DNA- coated tungsten or gold particles (Agracetus Inc., WI, USA) amongst others.

Alternatively, an expression construct of the invention is a viral vector. Suitable viral vectors are known in the art and commercially available. Conventional viral-based systems for the delivery of a nucleic acid and integration of that nucleic acid into a host cell genome include, for example, a retroviral vector, a lentiviral vector or an adeno-associated viral vector. Alternatively, an adenoviral vector is useful for introducing a nucleic acid that remains episomal into a host cell. Viral vectors are an efficient and versatile method of gene transfer in target cells and tissues. Additionally, high transduction efficiencies have been observed in many different cell types and target tissues.

For example, a retroviral vector generally comprises cis-acting long terminal repeats (LTRs) with packaging capacity for up to 6-10 kb of foreign sequence. The minimum cis-acting LTRs are sufficient for replication and packaging of a vector, which is then used to integrate the expression construct into the target cell to provide long term expression. Widely used retroviral vectors include those based upon murine leukemia virus (MuLV), gibbon ape leukemia virus (GaLV), simian immunodeficiency virus (SrV), human immunodeficiency virus (HIV), and combinations thereof (see, e.g., International publication WO1994/026877; Buchschacher & Panganiban, 1992; Johann et al., 1992; Sommerfelt & Weiss, 1990; Wilson et al., 1989; Miller et al., 1991; Lynch, et al., 1991; Miller & Rosman, 1989; Miller, 1990; Scarpa et al., 1991; Burnset al., 1993.

Various adeno-associated virus (AAV) vector systems have also been developed for nucleic acid delivery. AAV vectors can be readily constructed using techniques known in the art. (see, e.g., US patents 5173414 and 5139941; International publications WO 92/01070 and WO 93/03769; Lebkowski et al., 1988; Vincent et al., 1990; Carter, 1992; Muzyczka, 1992; Kotin, 1994; Shelling & Smith, 1994; Zhou et al., 1994.

Additional viral vectors useful for delivering an expression construct of the invention include, for example, those derived from the pox family of viruses, such as vaccinia virus and avian poxvirus or an alphavirus or a conjugate virus vector (e.g., that described in Fisher-Hoch et al., 1989.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

### Hematopoietic cells

In some examples the recipient subject will have received a donor graft comprising hematopoietic cells. In further examples, the donor graft is bone marrow or peripheral blood mononuclear cells (PBMC) harvested from the blood. Such grafts will comprise hematopoietic cells. Terms such as graft, bone marrow transplant, PBMC transplant are used to describe transplants containing hematopoietic cells. Hematopoietic cell transplantation (HCT) is an important and potentially curative treatment option for a wide variety of malignant and nonmalignant diseases. The multipotent hematopoietic stem cells required for this procedure are usually obtained from the bone marrow or peripheral blood of a related or unrelated donor. Umbilical cord blood, the blood remaining in the umbilical cord and placenta following the birth of an infant, has emerged as an established alternative source of hematopoietic stem cells in allogeneic HCT.

The term "hematopoietic cells" as referred to herein is a general term referring to progenitor/hematopoietic stem cells from any source (e.g, bone marrow, peripheral blood, cord blood). Otherwise, the source of such cells will be specified (eg, autologous peripheral blood progenitor cell transplantation).

In some examples, the PBMCs are obtained by apheresis of the donor. In this context, the donor may be an unrelated donor, in which case the graft is referred to as an allogeneic PBPC graft. In other examples, the PBMCs are obtained from the recipient prior to receiving treatment (e.g. chemotherapy treatment). In this context, the graft is referred to as an autologous PBPC graft.

In some examples, the PBPCs are obtained after the donor (ore recipient) has received a course of granulocyte colony stem cell factor (G-CSF) injections. Without wishing to be bound by theory, typically the subject will receive daily sub-cutaneous injections of G-CSF and their leukocyte count will be monitored every few days to monitor stem cell mobilisation into the peripheral blood. In some examples, a subject will have received consecutive daily injections of G-CSF for at least 7 days. In some examples, a subject will have received consecutive daily injections of G-CSF for at least 10 days. In some examples, G-CSF may be combined with another agent (e.g. plerixafor injection or stem cell factor (SCF)).

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

### Examples

### Example 1 Materials and methods

### Mesenchymal Lineage Precursor or Stem Cells (MLPSCs) prepared using plastic adherence techniques

MLPSCs were generated de novo from bone marrow as described in US 5,837,539. Approximately 80-100 ml of bone marrow was aspirated into sterile heparin-containing syringes and taken to the MDACC Cell Therapy Laboratory for MSC generation. The bone marrow mononuclear cells were isolated using ficoll-hypaque and placed into twoT175 flask with 50 ml per flask of MLPSC expansion medium which includes alpha modified MEM (αMEM) containing gentamycin, glutamine (2 mM) and 20% (v/v) fetal bovine serum (FBS) (Hyclone).
The cells were cultured for 2-3 days in 37°C, 5%CO₂ at which time the non-adherent cells were removed; the remaining adherent cells were continually cultured until the cell confluence reached 70% or higher (7-10 days), and then the cells were trypsinized and replaced in six T175 flasks with expansion medium (50 ml of medium per flask).

### Immunoselection of Mesenchymal Lineage Precursor or Stem Cells (MLPSCs)

Bone marrow (BM) was harvested from healthy normal adult volunteers (20-35 years old). Briefly, 40 ml of BM is aspirated from the posterior iliac crest into lithium-heparin anticoagulant-containing tubes.

Bone marrow mononuclear cells (BMMNC) were prepared by density gradient separation using Lymphoprep^{™} (Nycomed Pharma, Oslo, Norway) as previously described by Zannettino et al., 1998. Following centrifugation at 400 × g for 30 minutes at 4°C, the buffy layer is removed with a transfer pipette and washed three times in "HHF", composed of Hank's balanced salt solution (HBSS; Life Technologies, Gaithersburg, MD), containing 5% fetal calf serum (FCS, CSL Limited, Victoria, Australia).

STRO-3+ (or TNAP+) cells were subsequently isolated by magnetic activated cell sorting as previously described by Gronthos & Simmons, 1995; and Gronthos, 2003. Briefly, approximately 1-3 × 10⁸ BMMNC are incubated in blocking buffer, consisting of 10% (v/v) normal rabbit serum in HHF for 20 minutes on ice. The cells are incubated with 200 µl of a 10 µg/ml solution of STRO-3 mAb in blocking buffer for 1 hour on ice. The cells are subsequently washed twice in HHF by centrifugation at 400 × g. A 1/50 dilution of goat anti-mouse y-biotin (Southern Biotechnology Associates, Birmingham, UK) in HHF buffer is added and the cells incubated for 1 hour on ice. Cells are washed twice in MACS buffer (Ca²⁺ - and Mg²⁺ -free PBS supplemented with 1% BSA, 5 mM EDTA and 0.01% sodium azide) as above and resuspended in a final volume of 0.9 ml MACS buffer.

One hundred µl streptavidin microbeads (Miltenyi Biotec; Bergisch Gladbach, Germany) are added to the cell suspension and incubated on ice for 15 min. The cell suspension is washed twice and resuspended in 0.5 ml of MACS buffer and subsequently loaded onto a mini MACS column (MS Columns, Miltenyi Biotec), and washed three times with 0.5 ml MACS buffer to retrieve the cells which did not bind the STRO-3 mAb (deposited on 19 December 2005 with American Type Culture Collection (ATCC) under accession number PTA-7282 - see International publication WO 2006/108229). After addition of a further 1 ml MACS buffer, the column is removed from the magnet and the TNAP+ cells are isolated by positive pressure. An aliquot of cells from each fraction can be stained with streptavidin-FITC and the purity assessed by flow cytometry.

### Measurement of IL-2Rα expression

### Preparation of Culture Medium

DMEM containing 10% foetal bovine serum and 2 mM GlutaMax-I was prepared and filtered through a 0.2µm filter and labelled with a 1 month expiration date at 2-8°C. The culture medium was pre-warmed in a 37°C water bath for a minimum of 30 minutes prior to use.

### Preparation of 0.5% Trypsin/EDTA

EDTA containing 0.5% EDTA was prepared and filtered through a 0.2µm filter and labelled with a 12 month expiration date or reagent expiration date, whichever is sooner, and stored in aliquots at -20°C.

### Thawing MLPSCs

MLPSC batch sample vial was thawed. A maximum of 2 samples per batch were thawed. Sample vial was retrieved form the Vapor Phase LN₂ Freezer and transported on dry ice if thawed within ≤15 minutes or placed on wet ice if thawed immediately (≤1 minute). Sample vial was placed in the 37°C ± 2°C water bath and thawed for ≤5 minutes, removed from water bath, the outer surface sprayed or wiped with 70% isopropyl alcohol and transferred to a biosafety cabinet. Cells were transferred to a 50ml centrifuge tube with 15ml of pre-warmed culture medium drop wise using a 10ml syringe fitted with a 16g or larger needle and mixed well. The cells were subsequently passed through a 40µm cell strainer and collected in a new 50ml tube. The cells were then centrifuged at 250 × g at RT or 2-8°C for 8 minutes. The supernatant was discarded and the cells resuspended in 8 ml of culture medium (3-5 × 10⁶ cells/ml target concentration). The cells were titurated gently to ensure uniform suspension. 0.2ml sample was used to perform a trypan blue cell count. Average viability was ≥70%. The above procedure was repeated if two samples were tested.

### MLPSC pre-culture

Cells were seeded at 6,857 cells/cm² cells in a minimum of 3, preferably 4 × T175 flasks. Flasks were gently rocked to evenly distribute cells and subsequently incubated at 37°C ± 2°C, 5 ± 2% CO₂ overnight up to 24 hours. Cells were removed from the incubator and examined under a microscope to ensure ≥30% confluence, and observe the cell morphology for visible signs of contamination. The medium from each flask was aseptically aspirated and replaced with 30mL pre-warmed culture medium. The flasks were incubated at 37°C ± 2°C, 5 ± 2% CO₂ for a total of 60-84 hours from incubation start time on day one.

### Preparation of Co-culture Medium

Co-culture medium was prepared by adding equal volumes of culture medium and DMEM. The co-culture medium was pre-warmed in a 37°C water bath for a minimum of 30 minutes prior to use.

### Thawing PBMC

Qualified PBMC vial was thawed. The number of vials thawed depends on the total number of cells per vial (based on manufacturer's claim). If >2 × 10⁷ viable cells per vial, only one vial was thawed. Vial(s) was retrieved form the Vapor Phase LN₂ Freezer and transported on dry ice if thawed within ≤15 minutes or placed on wet ice if thawed immediately (≤1 minute). Vial was placed in the 37°C ± 2°C water bath. and thawed for 2-3 minutes, removed from water bath, the outer surface sprayed or wiped with 70% isopropyl alcohol and transferred to a biosafety cabinet. Cells were transferred to a 50ml centrifuge tube with 20mL of pre-warmed culture medium drop wise using a 10ml syringe fitted with a 16g or larger needle and mixed well. The vial was rinsed with culture medium and any residual cells added to the tube. The cells were subsequently passed through a 40µm cell strainer and collected in a new 50mL tube. The cells were then centrifuged at 350 × g at RT or 2-8°C for 5 minutes. The supernatant was discarded and the cells resuspended in co-culture medium at 2.5 × 10⁶ cells/ml.. 0.2ml sample was used to perform a trypan blue cell count. Average viability was ≥70%. 8ml of PBMC at 2 × 10⁶ cells/ml (1.6 × 10⁷ total PBMC) was prepared in co-culture medium and the PBMC incubated on ice while the MLPSCs were prepared. If total viable cells after cell count was <1.6 × 10⁷, another vial of PBMC was thawed and the above procedure repeated.

### Preparation of MLPSCs

Mesenchymal lineage cells were removed from the incubator and examined under a microscope to observe cell adherence and cell morphology (long and flat fibroblast). The medium from the flask was aseptically aspirated and cells washed with 9ml pre-warmed DPBS. The DPBS was subsequently aspirated and 4ml pre-warmed 0.05% trypsin/EDTA added per flask. The flasks were rocked to coat and incubated at 37°C for 3-6 minutes. The sides of the flasks were tapped during incubation. Cells were examined under the microscope to ensure that they were completely detached. If not detached, the flasks were tapped again. 7ml pre-warmed culture medium was added to each flask to dislodge cells. The cells from the flasks were pooled into a 50ml conical tube. The flasks were rinsed with an additional 1ml pre-warmed culture medium and any residual cells added to the tube. The cells were subsequently centrifuged at 350 × g at RT or 2-8°C for 5 minutes. The supernatant was aspirated and the cells resuspended in 2ml co-culture medium/flask. 0.2ml sample was used to perform a trypan blue cell count. Average viability was ≥70%. 1.5ml of MLC at 4 × 10⁵ cells/ml (6 × 10⁵ total MPC) was prepared in co-culture medium and set aside while the PBMC were stimulated.

### Stimulation of PBMC

5ml of PBMC suspension was transferred to a 15ml conical tube and 20µl of 1mg/ml CD3 antibody stock and 20µl of 1mg/ml CD28 antibody stock added to achieve final concentration of 4µg/ml anti-CD3 antibody and 4µg/ml anti-CD28 antibody (stimulated PBMC). The remaining PBMC were not stimulated. A 24-wel plate layout was followed as shown below.

| | 1 | 2 | 3 | 4 | | 5 | 6 |
|---|---|---|---|---|---|---|---|
| A | | | | | | Sample 1:_________ Co-Culture 500µl stimulated PBMC + 500µl ceMSCs | |
| B | | 500µl unstimulated PBMC + 500µl Co-Culture media | | 500µl stimulated PBMC + 5001µl Co-Culture media | | | |
| C | | | | | | □ N/A Sample 2: Co-Culture - 500µl stimulated PBMC + 500µl ceMSCs | |
| D | | | | | | | |

500µl unstimulated PBMC (negative control) was added to wells B2, B3, C2 and C3 of a 24 well plate. 500µl stimulated PBMC (positive control) was added to wells B4 and C4. 500µl stimulated PBMC was added to wells A5, B5, C5 and D5. If only one MLPSC sample was tested, 500µl stimulated PBMC was added to wells A5 and B5 only. 500µl culture expanded MLPSC (sample 1) was added to co-culture wells A5 and B5. 500µl culture expanded MLPSC (sample 2) was added to co-culture wells C5 and D5. 500µl co-culture medium was added to wells B2, B3, B4, C2, C3 and C4. All empty wells were filled with 1000µl co-culture medium to reduce evaporative loss. These conditions ware representative of a ratio of approximately 1 MLC: 5 PMBC.

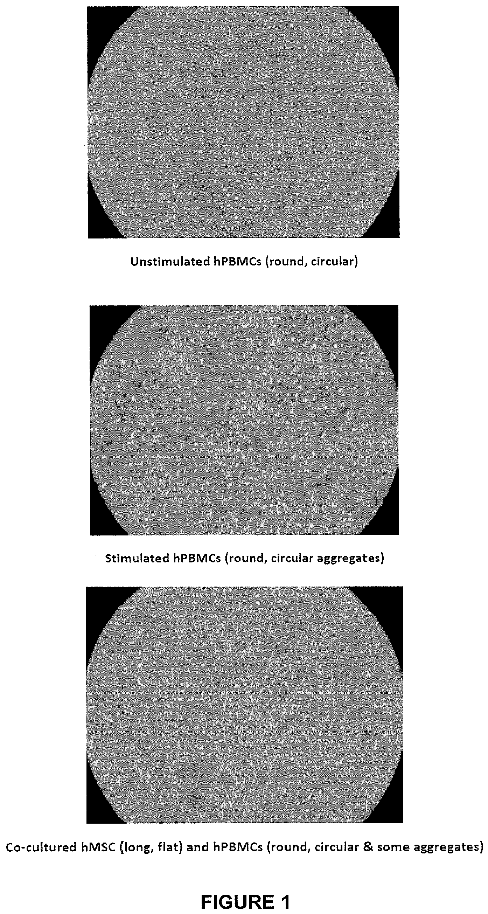

The plate was incubated at 37°C ± 2°C, 5 ± 2% CO₂ for 60-84 hours and morphology observed (see Figure 1).

### ELISA

IL-2Rα expression was measured by a commercially available ELISA kit, according to the manufacturer's instructions (R&D systems). The ELISA was performed following the manufacturer's protocol. The assay employs the quantitative sandwich enzyme immunoassay technique. The assay employs a microplate that includes wells that have been pre-coated with a monoclonal antibody specific for IL-2Rα. IL-2Rα present in calibrator samples, quality control samples, or co-cultured samples is captured by the immobilized IL-2Rα antibody. After washing away any unbound substances, enzyme-linked polyclonal antibodies specific for IL-2Rα is added to the wells. Following a wash step to remove any unbound enzyme-linked antibody, a substrate solution was added to the wells, and color develops in proportion to the amount of bound IL-2Rα. The color development is then stopped, and the intensity of the color is measured using an ELISA reader. The details of the ELISA are provided below.

The cells in each well were harvested using a 1000µl pipette and transferred to their respective microcentrifuge tubes. The wells were rinsed with 200µl pre-warmed DPBS and any residual cells added to the tubes. The culture plate was observed under the microscope to verify that the cells were completely removed. The cells were centrifuged at max rpm at RT or 2-8°C for 90 seconds. The supernatants were aspirated and the cell pellets placed on ice while the lysis buffer was prepared. The lysis buffer was prepared by adding one complete Mini-Tablet to 10ml CellLytic-M cell Lysis Extraction Reagent. The lysis buffer was vortexed to mix and stored on ice. 250µl lysis buffer was added to each tube. Each pellet was resuspended using a 1000µl pipette and vortexed. The cells were incubated on ice for 15 minutes. Duplicate lysates were then pooled and mixed. The lysates were centrifuged at max rpm at RT or 2-8°C for 10 minutes and the lysates transferred to new tubes, avoiding any pelleted materials. The lysates were stored for up to 29 days at ≤-60°C or the ELIZA performed on the same day. If the ELIZA was performed on the same day, the lysates were first frozen on dry ice or at ≤-60°C freezer for a minimum of 15 minutes.

All samples and reagents were brought up to ambient temperature and allowed to sit for at least 30 minutes before use. 20ml of concentrate wash buffer was diluted in 480ml NANO water (to give a 1:25 dilution) and mixed well. The solution was labelled with a 1 month expiration date or the expiration date of the kit, whichever is sooner and stored at 2-8°C. Triplicate wells were assigned for the standards, controls and samples on a microplate. Excess strips were removed from the microplate. The lysis buffer was prepared by adding two complete Mini-Tablets to 20 ml CellLytic-M Reagent. The lysis buffer was vortexed to mix and stored on ice. 1ml lysis buffer was added to two IL-2Rα standards to produce a 5000 pg/ml standard. The standards were incubated at RT with gentle agitation for a minimum of 30 minutes. After incubation, the volume of each standard was pooled and serial dilutions and control dilutions prepared according to Tables 1 and 2 below:

**Table 1: Serial Dilutions**

| Standard Dilutions | | | |
|---|---|---|---|
| Initial Concentration (pg/ml) | Volume of Standard (µl) | Volume of Lysis Buffer (µl) | Final Concentration (pg/ml) |
| 5000 | 400 | N/A | 5000 |
| 5000 | 400 | 400 | 2500 |
| 2500 | 400 | 400 | 1250 |
| 1250 | 400 | 400 | 625 |
| 625 | 400 | 400 | 313 |
| 313 | 400 | 400 | 156 |
| 156 | 400 | 400 | 78 |
| N/A | N/A | 750 | 0 |

**Table 2: Control Dilutions**

| Control Dilutions | | | | |
|---|---|---|---|---|
| Initial Concentration (pg/ml) | Volume of Standard (µl) | Volume of Lysis Buffer (µl) | Control | Final Concentration (pg/ml) |
| 5000 | 300 | 75 | 1 | 4000 |
| 5000 | 150 | 225 | 2 | 2000 |
| 5000 | 90 | 910 | 3 | 450 |

Unstimulated samples were prepared as per Table 3 below:

**Table 3: Unstimulated Samples (Negative control)**

| **PBMC lot No.** | Initial Non-Stimulated PBMC | Vol of Un-spiked Sample (µl) | Volume of 5000 pg/ml Std (µl) | Final Spike Concentration (pg/ml) | Final Sample Dilution |
|---|---|---|---|---|---|
| | 1x | 273 | 27 | 450 | ~1X |
| | 1x | 273 | 27 | 450 | ~1X |

Co-culture samples were prepared as per Table 4 below:

**Table 4: Co-culture Samples**

| **Sample ID** | Initial Dilution | Volume of ceMSCs/hPBMC lysate (µl) | Volume of Lysis Buffer (µl) | Final Sample Dilution |
|---|---|---|---|---|
| | 1x | 50 | 200 | 5X |
| | 1x | 50 | 200 | 5X |
| | 1x | 50 | 200 | 5X |
| | 1x | 50 | 200 | 5X |

100µl Assay Diluent RD1-1 was added to all wells of a microplate pre-coated with an anti-IL-2Rα monoclonal antibody. 50µl of standards, controls or samples was added to appropriate wells. Polyclonal anti- IL-2Rα HRP conjugate was added to each well and the plate was incubated covered for 3 hours ± 20 minutes at RT on an orbital shaker with gentle shaking. The plate was then washed 4 times with 300µl Wash buffer per well. After the last wash, residual liquid was removed by blotting the plate onto absorbent paper. Within 15 minutes of use, Substrate solution was prepared by mixing equal parts of Reagent A and Reagent B. 200µl substrate solution was added to each well, the plate covered and incubated in the dark for 20 ± 5 minutes at RT. 50µl Stop solution was added to each well and the optical density (OD) of each sample read on a microplate reader set to 450 nm with wavelength correction at 570 nm within 5 to 30 minutes. A standard curve was constructed using a four parameter logistic curve fit. The concentration of IL-2Rα in each sample was derived from the standard curve and corrected for dilutions to obtain a final result.

### Measurement of TNFR1 expression

### Preparation of Culture Medium

DMEM containing 10% foetal bovine serum and 1 mM GlutaMax-I was prepared and filtered through a 0.2µm filter and labelled with a 1 month expiration date at 2-8°C. The culture medium was pre-warmed in a 37°C water bath for a minimum of 30 minutes prior to use.

### Preparation of 0.5% Trypsin/EDTA

EDTA containing 0.5% EDTA was prepared and filtered through a 0.2µm filter and labelled with a 12 month expiration date or reagent expiration date, whichever is sooner, and stored in aliquots at -20°C.

### Thawing mesenchymal lineage cells

MLPSC batch sample vial was thawed. A maximum of 2 samples per batch were thawed. Sample vial was retrieved form the Vapor Phase LN₂ Freezer and transported on dry ice if thawed within ≤ 15 minutes or placed on wet ice if thawed immediately (≤ 1 minute). Sample vial was placed in the 37°C ± 2°C water bath. and thawed for ≤5 minutes, removed from water bath, the outer surface sprayed or wiped with 70% isopropyl alcohol and transferred to a biosafety cabinet. 2ml cells were transferred to 50ml centrifuge tube with 8mL of prewarmed culture medium drop wise using a 10ml syringe fitted with a 16g or larger needle and mixed well. The cells were subsequently passed through a 40µm cell strainer and collected in a new 50 mL tube. The cells were then centrifuged at 250 x g at RT or 2-8°C for 5 minutes. The supernatant was discarded and the cells resuspended in 5 mL of culture medium (3-5 x 10⁶ cells/mL target concentration). The cells were titurated gently to ensure uniform suspension. 0.2 ml sample was used to perform a trypan blue cell count. Average viability was ≥70%. The above procedure was repeated if two samples were to be tested.

### ELISA

TNFRI expression was measured by a commercially available ELISA kit, Quantikine, according to the manufacturer's instructions (R&D systems). The ELISA was performed following the manufacturer's protocol. This assay provides for the measurement of both soluble as well as cell-associated TNFRI (Qjwang et al., 1997). The assay employs the quantitative sandwich enzyme immunoassay technique. The assay employs a microplate that includes wells that have been pre-coated with a monoclonal antibody specific for TNFRI. TNFRI present in calibrator samples, quality control samples, or samples of MPC cell lysates is captured by the immobilized TNFRI antibody. After washing away any unbound substances, enzyme-linked polyclonal antibodies specific for TNFRI is added to the wells. Following a wash step to remove any unbound enzyme-linked antibody, a substrate solution was added to the wells, and color develops in proportion to the amount of bound TNFRI. The color development then is stopped, and the intensity of the color is measured using an ELISA reader. The details of the ELISA are provided below.

Cells were centrifuged at 250 x g at RT or 2-8°C for 5 minutes. The supernatant was aspirated and the cell pellet placed on ice while the lysis buffer was prepared. The lysis buffer was prepared by adding two complete Mini-Tablets to 20ml CellLytic-M cell Lysis Extraction Reagent. The lysis buffer was vortexed to mix and stored on ice. Lysis buffer was added. Each pellet was resuspended using a 1000µl pipette and vortexed. The cells were incubated on ice for 10-15 minutes and vortexed every 5 minutes.. Duplicate lysates were then pooled, mixed and transferred to 2ml microcentrifuge tube(s). The lysates were centrifuged at max rpm at 2-8°C for 10 minutes and the lysates transferred to new tubes, avoiding any pelleted materials. The lysates were stored for up to 29 days at ≤-60°C or the ELIZA performed on the same day. If the ELIZA was performed on the same day, the lysates were first frozen on dry ice or at ≤-60°C freezer for a minimum of 1 hour.

All samples and reagents were brought up to ambient temperature and allowed to sit for at least 30 minutes before use. 20ml of concentrate wash buffer was diluted in 480ml NANO water (to give a 1:25 dilution) and mixed well. The solution was labelled with a 1 month expiration date or the expiration date of the kit, whichever is sooner and stored at 2-8°C. Triplicate wells were assigned for the standards, controls and samples on a microplate. Excess strips were removed from the microplate. The lysis buffer was prepared by adding two complete Mini-Tablets to 20 ml CellLytic-M Reagent. The lysis buffer was vortexed to mix and stored on ice. 0.5ml lysis buffer was added to sTNFR1 standard to produce a 5000 pg/ml standard. The standard was incubated at RT with gentle agitation for a minimum of 15 minutes. After incubation, serial dilutions and control dilutions prepared according to Tables 5 and 6 below:

**Table 5: Serial Dilutions**

| Standard Dilutions | | | |
|---|---|---|---|
| Initial Concentration (pg/ml) | Volume of Standard (µl) | Volume of Lysis Buffer (µl) | Final Concentration (pg/ml) |
| 5000 | 150 | 1350 | 500 |
| 500 | 750 | 750 | 250 |
| 250 | 750 | 750 | 125 |
| 125 | 750 | 750 | 62.5 |
| 62.5 | 750 | 750 | 31.3 |
| 31.2 | 750 | 750 | 15.6 |
| 15.6 | 750 | 750 | 7.8 |
| 0 | N/A | 650 | 0 |

**Table 6: Control Dilutions**

| Control Dilutions | | | | |
|---|---|---|---|---|
| Initial Concentration (pg/ml) | Volume of Standard (µl) | Volume of Lysis Buffer (µl) | Control | Final Concentration (pg/ml) |
| 5000 | 18 | 1982 | 1 | 45 |
| 5000 | 40 | 1960 | 2 | 100 |
| 5000 | 1 00 | 1 900 | 3 | 250 |

Samples were prepared as per table 7 below:

**Table 7: Samples**

| Sample ID# | Vol of 5000 pg/ml Standard (µl) | Initial Sample Dilution | Vol of Sample (µl) | Final Spike Concentration (pg/ml) |
|---|---|---|---|---|
| | 13 | 1X | 637 | 100 |
| | 13 | 1X | 637 | 100 |
| | 13 | 1X | 637 | 100 |
| | 13 | 1X | 637 | 100 |

50µl Assay Diluent HD1-7 was added to all wells of a microplate pre-coated with an anti-TNFR1 monoclonal antibody. 200µl of standards, controls or samples was added to appropriate wells. The plate was covered and incubated for 2 hours ± 10 minutes at RT on an orbital shaker with gentle shaking. The plate was then washed 3 times with 300µl Wash buffer per well. After the last wash, residual liquid was removed by blotting the plate onto absorbent paper. Polyclonal anti-TNFR1 HRP conjugate was added to each well and the plate was incubated covered for 2 hours ± 10 minutes at RT on an orbital shaker with gentle shaking. The plate was then washed 3 times with 300µl Wash buffer per well. After the last wash, residual liquid was removed by blotting the plate onto absorbent paper. Within 15 minutes of use, Substrate solution was prepared by mixing equal parts of Reagent A and Reagent B. 200µl substrate solution was added to each well, the plate covered and incubated in the dark for 20 ± 10 minutes at RT. 50µl Stop solution was added to each well and the optical density (OD) of each sample read on a microplate reader set to 450 nm with wavelength correction at 570 nm within 5 to 30 minutes. A standard curve was constructed using a four parameter logistic curve fit. The concentration of TNFR1 in each sample was derived from the standard curve and corrected for dilutions to obtain a final result.

### Statistical analysis

Objective assessment of the response of acute GVHD (aGVHD) to treatment with MSCs or MPCs was determined as the overall response rate at day 28. To present changes in aGVHD organ stage, response data from baseline to day 28 was classified for each organ as complete response, partial response, worsening, or stable.

To evaluate the effect of response on overall survival, two Kaplan-Meier survival analyses were conducted through day 100. A Kaplan-Meier curve was generated for patients who had achieved overall response (complete response or partial response) at day 28 and another Kaplan Meier curve was generated for nonresponders at day 28. The null hypothesis of no difference in overall survival between the 2 groups was tested with the log-rank test using commercial software. The testing was performed at a significance level of p< 0.05.

Categorical variables were summarised as frequencies and percentages. Continuous variables were summarised using descriptive statistics (number, mean, standard deviation, median and range). All confidence intervals had a 95% confidence level.

### Example 2: Immunoselected mesenchymal lineage precursor or stem cells with enhanced immunosuppressive properties

The immunosuppressive potential of immunoselected mesenchymal lineage precursor or stem cells (MLPSCs) were assess by comparison with products obtained by the previous manufacturing process (as described in US 9,828,586). Results of the T cell proliferation assay (% inhibition of IL2R ) performed on three different samples of MLPSCs produced under the previous manufacturing conditions (i.e. samples MLPSC A, MLPSC B and MLPSC C) and three different samples of improved immunoselected MLPSCs with (i.e. samples MLPSC D, MLPSC E and MLPSC F) compared to are shown in Figure 2. These results show that the assay differentiates between a first class of multipotential lineage cells and a second class of multipotential lineage cells in terms of ability to inhibit T-cell proliferation. In particular, the second class of cells inhibits T cell proliferation substantially more effectively that the first class.

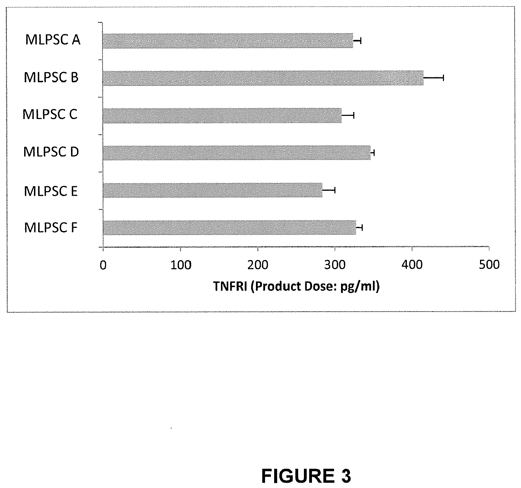

In contrast, results of the TNFR1 expression assay as shown in Figure 3 showed no significant differentiation between the same two classes of mesenchymal lineage precursor or stem cells. TNFR1 expression has previously been described as a marker for the identification of cells that inhibit T cell proliferation (see US20140248244).

Results presented herein in Figures 2 and 3 show that it is possible to produce improved mesenchymal lineage precursor or stem cell populations with enhanced ability to inhibit T-cell proliferation. These improved mesenchymal lineage precursor or stem cell populations are a subset of cells that express high levels of TNFR1. In other words, TNFR1 expression can be dissociated from the T cell inhibitory properties of certain mesenchymal lineage precursor or stem cell populations. The fact that it is possible to produce mesenchymal lineage precursor or stem cell populations that exhibit enhanced ability to inhibit T-cell proliferation even after cryopreservation and thawing is particularly surprising in light of the conventional wisdom that that cryopreserved mesenchymal stem cells display impaired immunosuppressive properties following thawing (Francois et al., 2012; Chinnadurai et al., 2016).

### Example 3: Example 4: Mesenchymal lineage precursor or stem cell (MLPSC) with low IL-2R inhibition infused for the treatment of pediatric subjects who are steroid refractory

This study is for the treatment of pediatric patients who have failed to respond to steroid treatment for acute graft-versus-host disease (GVHD). Failing steroid treatment for acute GVHD is defined as any Grade B-D (IBMTR grading) of acute GVHD that is not improving after at least 3 days of methylprednisolone (≥ 1 mg/kg/day) or equivalent.

Patients were treated with ex-vivo cultured MLPSC with low IL-2R inhibition *twice* per week at a dose of 2 × 10⁶ hMSC/kg (actual body weight) for each of 4 consecutive weeks. Infusions were administered at least 3 days apart.

Study design:
241 pediatric patients undergoing HSCT were enrolled and treated at 50 sites in North America and Europe from 2007-2014
Ages 2 months - 17 years
Acute GvHD grades B-D (CIBMTR)
Failed steroid treatment and multiple other agents
aGVHD not improving after at least 3 days of methylprednisolone (at least 1 mg/kg/day or equivalent)

Results:
In 241 Children under EAP, Overall Response (CR+PR) at Day 28 was 65% (95% CI: 58.9%, 70.9%). Day 100 survival correlated with overall response, and was significantly improved in those who responded at Day 28 (82% vs. 39%, p<0.0001)

### Example 4: Mesenchymal lineage precursor or stem cell (MLPSC) with high IL-2R inhibition infused for the treatment of pediatric subjects who are steroid refractory (Low dose)

### Study objectives

The primary objective was to gather information of the safety of repeated doses of MLPSC with high IL-2R inhibition administered intravenously in subjects with Grade B-D aGVHD who have failed to respond to steroid treatment post allogeneic HSCT and to evaluate the efficiency of repeated doses of MPCs administered intravenously in subjects with Grades B-D aGVHD who have failed to respond to steroid treatment post allogeneic HSCT.

A secondary objective of the study was to determine the correlation between response to MPCs at Day28 and survival at Day 100.

### Treatment plan

Pediatric subjects treated with intravenous (IV) MPCs at a dose of 2 × 10⁶ MPC/kg (body weight at screening)* once per week for each of 4 consecutive weeks.

If eligible, the subject may receive an additional 4 once-weekly infusions of MPCs at a reduced dose of 1 × 10⁶ MPC/kg following the initial four doses. Eligibility to receive additional therapy was dependent upon the subject's acute GVHD (aGVHD) response assessment performed at Day 28 (partial or mixed).
At most, a subject received eight infusions.

### Acute GVDH assessments

Performed at screening, Day 14, Day 28, Day 56 and Day 100/end of study.

### Subject Demographics

Twelve (12) subjects were treated at seven transplant centres. Six of the subjects were treated at the Fed Hutchinson Cancer Research Centre with the remaining subjects each treated at a different transplant centre (as shown in Table 10).

All subjects were less than 18 years of age with a range between 3 and 17 years. The mean age was 10.3 years and the median age was 10.5 years.

Subjects who were eligible to participate in the trial must have failed to respond to steroid treatment for Grades B to D acute GVHD (aGVHD) following allogeneic haematopoietic stem cell treatment (HSCT). Failure to respond to steroid treatment for acute GVHD is defined as any Grade B-D acute GVHD that is not improving after at least three (3) days of methylprednisolone (>1 mg/kg/day) or equivalent.

All subjects had visceral organ disease, involving the lower gastro intestinal tract (GI) and/or liver. Nine subjects had lower GI alone (8 with Grade D, 1 with Grade B); one subject had Grade C lower GI and Grade D liver; one subject had Grade B upper GI and Grade C lower GI; and one subject had Grade C liver.

At baseline, nine of the twelve subjects (9/12 or 75%) had Grade D graft versus host disease (GVHD), two of twelve subjects (2/12 or 17%) had Grade C GVHD and one subject (1/12 or 18%) had Grade B GVHD.

Subjects were treated with a mean of 4.5 GVHD therapies prior to MPC treatment, including the following non-steroid therapies: Extracorporeal photopheresis (ECP; 7 subjects), infliximab (5 subjects), Ruxolitinib (3 subjects), mycophenolic acid (MMF; 3 subjects), Etanercept (1 subject) or Basiliximab (1 subject).

Subjects were treated intravenously (IV) at a dose of 2 × 10⁶ cells/kg based on the subject's body weight at screening. The cells were delivered to the subject once per week (qw) for each of four consecutive weeks.

Eligible subjects received an additional four once-weekly infusions of cells at a reduced dose of 1 × 10⁶ cells/kg following the initial four doses. Eligibility to receive additional therapy was dependent upon the subject's acute GVHD (aGVHD) response assessment performed at Day 28 (partial or mixed). At most, a subject received eight infusions.

GVHD assessment was performed at screening, Day 14, Day 28, Day 56 and Day 100 (end of study).

### Response to treatment and survival through 100 days

The response to treatment with MLPSC with high IL-2R inhibition (administered 2 x 10⁶ MPCs/kg body weight once per week) and survival of subjects is summarised in Table 9 below. The GVHD assessments were performed at screening (day 0), day 14, day 28, day 56 and day 100 after receiving cell infusions. Body weight for calculating of cell dosing is based on the body weight of the subject at screening.

Ten of twelve (10/12) subjects (83%) survived to day 100 with seven receiving eight infusions and three receiving four infusions. All 12 subjects had lower GI and/or liver GVHD with 9/12 subjects (75%) having Grade D GVHD at baseline and only 1 subject (8%) having grade B GVHD at baseline. Mean number of prior treatments that got acute GVHD was 4.25.

**Table 9: GVHD assessment in subjects to day 100**

| **Outcome** | **GVHD Assessment** | | | |
|---|---|---|---|---|
| **Clinical response** | **Day 14** | **Day 28** | **Day 56** | **Day 100** |
| Overall (CR + PR) | 7 (58%) | 9 (75%) | 9 (75%) | 9 (75%) |
| *Complete* | *2 (17%)* | 1 (8%) | 5 (42%) | 5 (42%) |
| *Partial* | 5 (42%) | *8 (67%)* | 4 (33%) | 4 (33%) |
| *Mixed* | 0 | 0 | 0 | 0 |
| No response- stable | 2 (17%) | 1 (8%) | 0 | 1 (8%) |
| No response-worsening | 2 (17%) | 0 | 1 (8%) | 0 |
| Death | 1 (8%) | 2 (17%) | 2 (17%) | 2 (17%) |

| | | | | |
|---|---|---|---|---|
| CR= complete response PR= partial response | | | | |

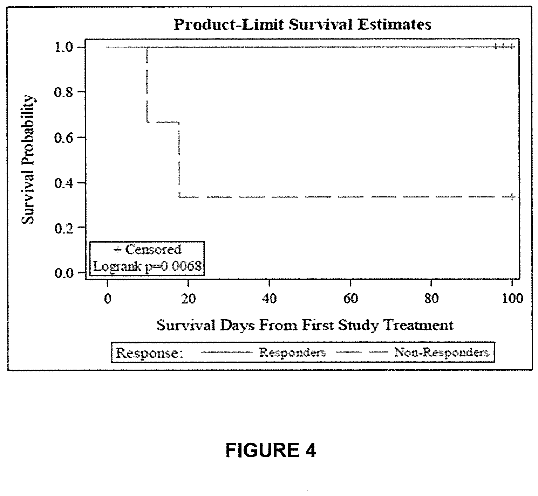

Figure 4 shows graphically the survival through 100 days for responders versus non-responders where survival probability is shown in the Y axis against survival days from first study treatment where Day 0 is baseline. All nine responders at Day 28 survived through to Day 100. In contrast one of three non-responders at Day 28 survived to Day 28 (i.e. 75% versus 8%).

The results show that overall response rate (i.e. complete and partial response rate) at day 28 was 75%. This is higher than the average overall response rate seen following infusion of MSCs. Furthermore, the response rate seen at day 28 was a strong predictor of overall survival at day 100 (see Table 9).

Notably, no identified safety issues were observed following infusion of MLPSC with high IL-2R inhibition.

The response to treatment with MLPSC with high IL-2R inhibition and survival of individual subjects in show in Table 10 provided below.

**Table 10: GVHD assessment in subjects**

| **Pt ID./age and Institution** | **GVHD Assessment** | | | | | **Total No. MPC infusions** |
|---|---|---|---|---|---|---|
| | **Baseline** | **Day 14** | **Day 28** | **Day 56** | **Day 100** | |
| *Fred Hutch* | | | | | | |
| 001 | Lower GI | CR | CR | CR | CR | 4 |
| 8 years | Grade D | | | | | |
| 002 | Lower GI | Died | Died | Died | Died | 2 |
| 3 years | Grade D | | | | | |
| 003 | Lower GI | PR | PR | PR | PR | 8 |
| 12 years | Grade D | | | | | |
| 004 | Lower GI | PR | PR | NR-worsening | CR | 4 |
| 15 years | Grade D | | | | | |
| 005 | Lower GI | CR | PR | CR | PR | 8 |
| 8 years | Grade B | | | | | |
| 006 | Lower GI | PR | PR | CR | CR | 8 |
| 11 years | Grade D | | | | | |
| *Cleveland Clinic* | Lower GI | NR-stable | NR-stable | PR | NR-stable | 4 |
| 16759 | Grade D | | | | | |
| 10 years | | | | | | |
| *Montefiore* | Lower GI | NR-stable | PR | CR | CR | 8 |
| 16896 | Grade D | | | | | |
| 17 years | | | | | | |
| *.Memorial* | Lower GI | NR-worsening | PR | PR | PR | 8 |
| *Sloan* | Grade C | | | | | |
| 17042 | and Liver | | | | | |
| 5 years | Grade D | | | | | |
| *Univ Pitts Medical Ctr* | Lower GI | PR | PR | PR | PR | 8 |
| Lower GI Grade D | Grade D | | | | | |
| *Duke Univ* | Liver | NR-worsening | Died | Died | | 1 |
| 17093 | Grade C | | | | | |
| 17 years | | | | | | |
| *UCSF Oakland* | Upper GI | PR | PR | CR | CR | 8 |
| | Grade B and Lower GI Grade C | | | | | |
| 17163 | | | | | | |
| 3 years | | | | | | |

| | | | | | | |
|---|---|---|---|---|---|---|
| CR= complete response PR= partial response | | | | | | |

No safety concerns were identified with the patients treated and the pilot study in GI tract disease demonstrated an overall response rate of 75% (Table 1). Of those subjects who responded at Day 28 following infusion of MLPSC with high IL-2R inhibition, 100% survived to Day 100. This indicates that the Day 28 response is indicative of a durable meaningful response and correlated with survival at Day 100.

Accordingly, the results show that comparable, if not superior response rates could be achieved in subjects receiving MLPSC (in this case MPCs) with high IL-2R inhibition at a ***once*** weekly dose which is half of that of the 2 × 10⁶ cells/kg body weight ***twice*** per week dosage required when administering MLPSC with low IL-2R inhibition (see comparative example 3 above).

### Example 5 Improved manufacturing process for MSCs

In view of the superior results obtained in the treatment of GvHD with MLPSC exhibiting high IL-2R inhibition (as described in Example 4) an investigation was made into processes for producing high potency MLPSC from stem cells isolated using plastic adherence techniques.

The previous manufacturing process for producing MLPSCs from cells isolated using plastic adherence techniques is described in US 9,828,586. A number of changes described below were introduced in this previous manufacturing process. Surprising, this improved manufacturing process (described below) resulted in the generation of MLPSCs with enhanced immunosuppressive characteristics.

### Equipment Changes

One equipment change relates to the method used to wash, transfer and concentrate cells. The previous process used the Cytomate Cell Processor (Baxter) at several different steps in the process for these activities. This piece of equipment is a benchtop device with associated disposables that was originally designed for washing, concentrating and transferring white blood cells. In the previous process, the Cytomate was used to seed cells into the culture vessels (Cell Factory) and at harvest it was used to wash and concentrate cells by volume reduction. In the new process, procedures for cell seeding (syringe trees) and the cell wash and concentration step (tangential flow filtration (TFF)) replaced the Cytomate.

### Other Changes to Process and Testing

The following aspects of the manufacturing and testing of an ceMSC product were also implemented in the improved manufacturing process.
- Use of Cell Factories with the air filter attached (pre-assembled) before sterilization to reduce aseptic processing risks.
- Use of recombinant trypsin, produced in yeast, was introduced to eliminate use of porcine trypsin to minimize risks from animal origin adventitious agents, such as parvovirus and circovirus. This required changes to trypsin incubation time with the cells and solutions used at this step.
- The use of a blood filter was introduced to reduce reduce/minimize potential for cell clumping and visible particulate matter.
- The use of cryovials as the final container and a 4.3 mL fill volume rather than cryobags and 15mL fill volume was introduced. The same concentration of cells/mL was maintained as for the previous process, but instead of a one cryobag, the equivalent number of cells is divided into four vials.

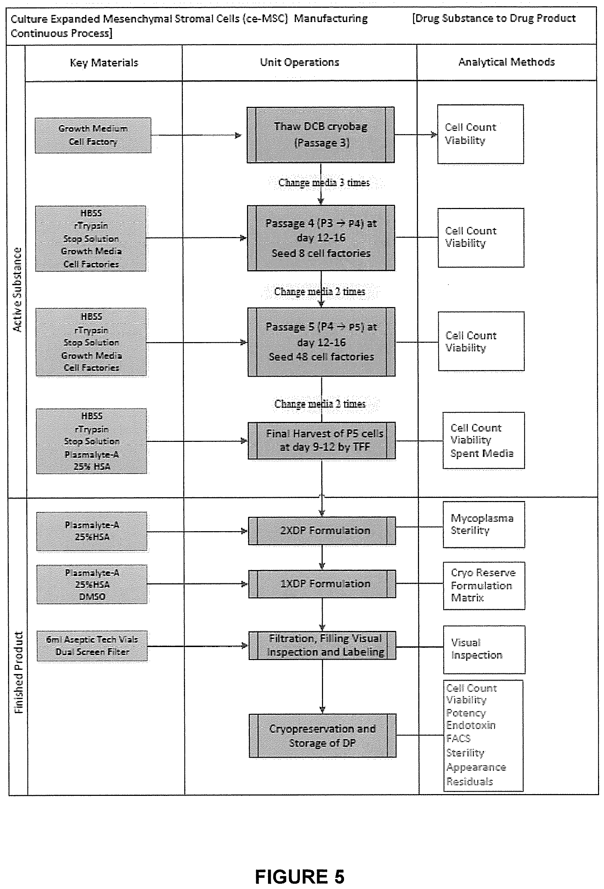

All final product testing is performed on samples of cryopreserved cells in the final product after thaw. Under the previous process, some testing was performed on cell aliquots stored in tubes not representative of the final container. Figure 5 shows all the steps from thaw of the donor cell bank (DCB) to cryopreservation and testing of the ce-MSC product.

### Example 6 - Testing MLPSC products obtained by improved manufacturing process

The MLPSC final product lots manufactured according to the improved manufacturing process described in Example 5 were tested for the release criteria set out in Table 8 below:

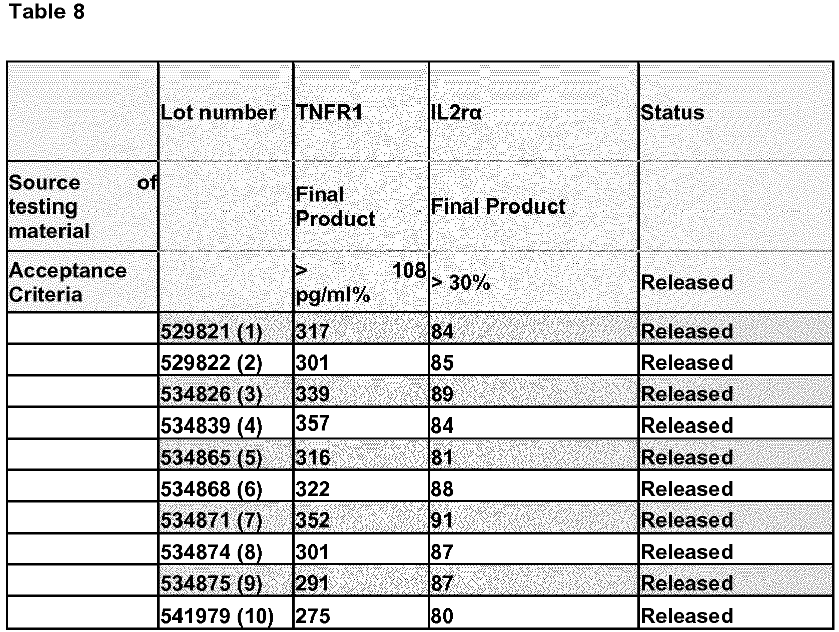

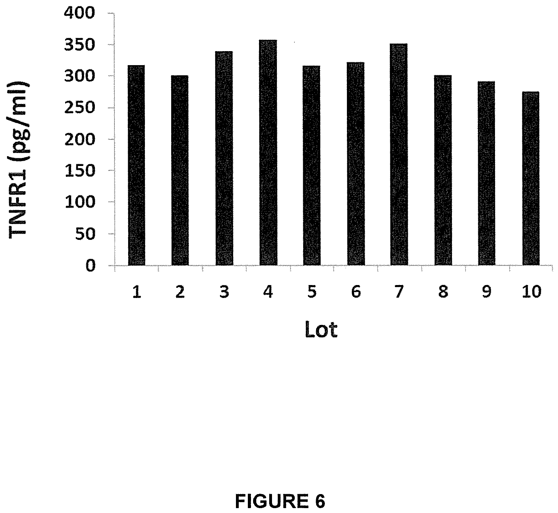

Table 8 shows consistently high levels of expression of TNFR1 by all of the product lots tested. As shown in Figure 6, each product lot showed TNFR1 expression levels of >275 pg/ml.

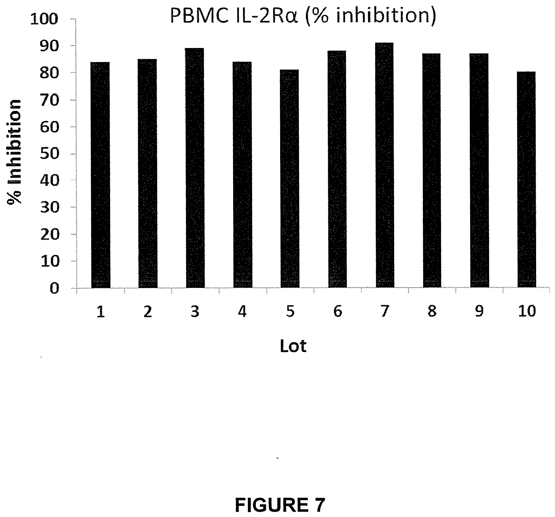

Table 8 also shows unexpectedly high levels of inhibition of IL-2Ra expression by the 10 product lots tested. As shown in Figure 7, each product lot showed inhibition levels of 80% or higher.

### References

Ausubel, F. M. (Ed.). (1987 including all updates untill present). Current Protocols in Molecular Biology. New York: John Wiley & Sons.
Brown, T. A. (Ed.). (1991). Essential Molecular Biology: A Practical Approach (Vol. 1 and 2). Oxford: IRL Press at Oxford University Press.
Buchschacher & Panganiban (1992). Journal of Virology, 2731-2739.
Burns et al., (1993). Proceedings of the National Academy of Sciences USA, 8033-8037.
Carter (1992). Current Opinion in Biotechnology, 533-539.
Chinnadurai et al., (2016). Translational and Clinical Research, 34(9), 2429-2442.
Coligan, J. E., Kruisbeek, A. M., Margulies, D. H., Shevach, E. M., & Strober, W. (Eds.). (1991 including all updates until present). Current Protocols in Immunology. New York: John Wiley & Sons.
Fisher-Hoch et al., (1989). Proceedings of the National Academy of Sciences USA, 56, 317-321.
Francois et al., (2012). Cytotherapy, 14(2), 147-152.
Glover, M., & Hames, B. D. (Eds.). (1995 and 1996). DNA Cloning: A Practical Approach (Vols. 1-4).
Gronthos (2003). Journal of Cell Science, 116(Pt 9), 1827-1835.
Gronthos & Simmons (1995). Blood, 85(4), 929-940.
Harlow, E., & Lane, D. (1988). Antibodies: A Laboratory Manual. New York: Cold Spring Harbor Laboratory Press.
Johann et al., (1992). Journal of Virology, 65, 1635-1640.
Kotin (1994). Human Gene Therapy, 793-801.
Lebkowski et al., (1988). Molecular and Cellular Biology, 3988-3996.
Miller (1990). Human Gene Therapy, 7, 5-14.
Miller & Rosman (1989). Biotechniques, 7, 980-990.
Miller et al., (1991). Journal of Virology, 65, 2220-2224.
Muzyczka (1992). Current Topics in Microbiology and Immunology, 158, 97-129.
Perbal, B. V. (1984). A Practical Guide to Molecular Cloning. New York: Wiley.
Qjwang et al., Biochemistry (1997) 36, 6033.
Sambrook, J., & Green, M. R. (2012). Molecular Cloning: A Laboratory Manual (Fourth Edition). New York: Cold Spring Harbour Laboratory Press.
Scarpa et al., (1991). Virology, 75, 849-852.
Schwartz et al., (2001). Nature 410, 604-608.
Schwartz et al., (2002). Nature Immunol. 3, 427-434.
Sommerfelt & Weiss (1990). Virology, 76, 58-59.
Vincent et al., (1990). Vaccine, 353-359.
Wilson et al., (1989). Journal of Virology, 63, 2374-2378.
Zannettino et al., (1998). Blood, 92(8), 2613-2628.
Zhang et al., (2004). Immunity, 20, 337-347.

The invention provides, in particular, the following:
1. A composition comprising mesenchymal lineage precursor or stem cells wherein the mesenchymal lineage precursor or stem cells are cryopreserved and, after thawing, inhibit proliferation of activated T cells in a sample of PBMC by at least about 65%.
2. The composition of item 1, wherein the inhibition of proliferation of activated T cells is measured by inhibition of IL-2R 2Rα expression in the activated T cells.
3. The composition of item 1 or item 2, wherein co-incubation of mesenchymal lineage precursor or stem cells with PMBC at a ratio of 1 mesenchymal lineage precursor or stem cell:5 PMBC, or less, inhibits T cell proliferation by at least about 65%.
4. The composition of item 1 or item 2, wherein co-incubation of mesenchymal lineage precursor or stem cells with PMBC at a ratio of 1 mesenchymal lineage precursor or stem cell:10 PMBC, or less, inhibits T cell proliferation by at least about 65%.
5. The composition of item 1 or item 2, wherein co-incubation of mesenchymal lineage precursor or stem cells with PMBC at a ratio of 1 mesenchymal lineage precursor stem cell:50 PMBC, or less, inhibits T cell proliferation by at least about 65%.
6. The composition of item 1 or item 2, wherein co-incubation of mesenchymal lineage stem cells with PBMC at a ratio of 1 mesenchymal lineage precursor or stem cell:100 PBMC, or less, inhibits T cell proliferation by at least about 65%.
7. The composition of any one of items 1 to 6, wherein co-incubation of mesenchymal lineage precursor or stem cells with PMBC at a ratio of 1 mesenchymal lineage precursor or stem cell:5 PMBC, or less, inhibits T cell proliferation by at least about 70%.
8. The composition of any one of items 1 to 7, wherein co-incubation of mesenchymal lineage precursor or stem cells with PMBC at a ratio of 1 mesenchymal lineage precursor or stem cell:5 PMBC, or less, inhibits T cell proliferation by at least about 80%.
9. The composition of any one of items 1 to 8, wherein the mesenchymal lineage precursor or stem cells express TNFR1 in an amount of at least 270 pg/ml.
10. The composition of any one of items 1 to 8, wherein the mesenchymal lineage precursor or stem cells express TNFR1 in an amount of at least 300 pg/ml.
11. The composition of any one of items 1 to 8, wherein the mesenchymal lineage precursor or stem cells express TNFR1 in an amount of at least 320 pg/ml.
12. The composition of any one of items 1 to 11, wherein the mesenchymal lineage precursor or stem cells are isolated by immunoselection.
13. The composition of any one of items 1 to 12, wherein the mesenchymal lineage precursor or stem cells are culture expanded mesenchymal stem cells.
14. A method of treating an inflammatory disorder in a subject in need thereof, comprising administering to the subject the composition comprising mesenchymal lineage precursor or stem cells according to any one of items 1 to 13.
15. The method according to item 14, wherein the inflammatory disorder is a T-cell mediated inflammatory disorder.
16. A method of preventing, alleviating the development of, or treating graft versus host disease (GVHD) in a subject, comprising administering to the subject the composition comprising mesenchymal lineage precursor or stem cells according to any one of items 1 to 13.
17. The method according to any one of items 14 to 16, wherein the composition is administered to the subject at a dose of less than 3 × 10⁶ cells/kg body weight once per week (qw).
18. The method according to any one of items 14 to 17, wherein the composition is administered to the subject at a dose of about 2 × 10⁶ cells/kg body weight qw.
19. The method according to any one of items 14 to 18 wherein the composition is administered to the subject at a maximal dose of 2 × 10⁶ cells/kg body weight qw.
20. The method according to any one of items 14 to 19, wherein the composition is administered as a single dose or as a divided dose(s).
21. A method for preventing , alleviating the development of, or treating graft versus host disease (GVHD) in a mammalian subject, comprising administering to the subject, mesenchymal lineage precursor or stem cells (MLPSCs) and/or progeny cells thereof at a dose of less than 3 × 10⁶ MPC/kg body weight once per week (qw).
22. The method according to item 21, wherein the subject is administered MLPSCs and/or progeny cells thereof at a dose of about 2 × 10⁶ cells/kg body weight qw.
23. The method according to item 21 or 22, wherein the subject is administered MLPSCs and/or progeny cells thereof at a maximal dose of 2 × 10⁶ cells/kg body weight qw.
24. The method according to any one of items 14 to 23, wherein the mammal is a pediatric human subject.
25. The method according to any one of items 14 to 24, wherein the subject has a malignant or genetic disorder of the blood.
26. The method according to any one of items 14 to 25, wherein the subject has received, is receiving or is about to receive a donor graft comprising hematopoietic cells.
27. The method according to item 26, wherein the graft comprises hematopoietic stem cells (HSCs).
28. The method according to any one of items 26 or 27, wherein the graft comprises allogeneic hematopoietic cells.
29. The method according to any one of items 14 to 28, wherein the MLPSCs are mesenchymal precursor cells (MPCs).
30. The method according to any one of items 14 to 28, wherein the MLPSCs and/or progeny cells thereof are administered beginning on the day of transplantation of the graft.
31. The method according to any one of items 14 to 28, wherein the MLPSCs and/or progeny cells thereof are administered after the subject has been determined to be steroid refractory.
32. The method according to any one of items 14 to 30, wherein the subject has acute GVHD.
33. The method according to any one of items 14 to 31, wherein the cells are administered in the form of a pharmaceutically acceptable composition.

## Claims

1. A method for selecting a population of mesenchymal lineage precursor or stem cells (MLPSCs) suitable for use in therapy comprising:
(i) obtaining a population of MLPSCs that has been culture expanded, cryopreserved and thawed,
(ii) co-culturing the obtained population of MLPSCs in a culture medium with a population of cells comprising T cells;
(iii) selecting a population of co-cultured MLPSCs which inhibit T cell IL-2Rα expression by ≥ 65%.

2. The method of claim 1, wherein culture expansion comprises:
- (a) culturing, in a cell factory with one or more attached air filters, a population of mesenchymal lineage precursor or stem cells in a culture medium comprising recombinant trypsin; and (b) reducing visible particulates and/or cell aggregates by
- (bi) concentrating and/or washing the cultured cells with tangential flow filtration (TFF); or
- (bii) passing the harvested cells through a dual screen mesh filter.

3. The method of claim 1 or 2, wherein the population of MLPSCs are obtained by immunoselection, wherein the immunoselected cells are STRO-1⁺.

4. The method of any one of claims 1 to 3, wherein the population of cells comprising T cells is a population of PBMCs.

5. The method of any one of claims 1 to 4, wherein the step of co-culturing comprises culturing MLPSCs and PBMCs at a ratio of 1 MLPSC:5 PMBC or less.

6. The method of claim 5, wherein the step of co-culturing comprises culturing MLPSCs and PBMCs at a ratio of:
- 1 MLPSC:10 PMBC, or less;
- 1 MLPSC:50 PMBC, or less; or,
- 1 MLPSC: 100 PMBC, or less.

7. The method of any one of claims 1 to 6, wherein the step of selecting comprises selecting a population of MLPSCs that inhibit T cell IL-2Rα expression by ≥ 70%.

8. The method of any one of claims 1 to 7, wherein the step of selecting comprises selecting a population of MLPSCs that inhibit T cell IL-2Rα expression by ≥ 80%.

9. A selected population of MLPSCs obtained by the method according to any one of claims 1 to 8.

10. A selected population of MLPSCs of claim 9 for use in a method of treating graft versus host disease (GvHD).

11. The selected population for use of claim 10, wherein the GvHD is steroid refractory.
